(19) Europäisches Patentamt
European Patent Office
Office européen des brevets

(11) Publication number: **0 172 632**
**B1**

(12) **EUROPEAN PATENT SPECIFICATION**

(45) Date of publication of patent specification: **30.08.89**

(21) Application number: **85304630.8**

(22) Date of filing: **28.06.85**

(51) Int. Cl.⁴: **C 07 D 491/06,**
A 61 K 31/415, C 07 D 311/86
// (C07D491/06, 311:00, 231:00)

(54) **Benzopyrano[4,3,2-cd]indazole compounds, processes for their production and pharmaceutical compositions comprising the compounds.**

(30) Priority: **29.06.84 US 626090**

(43) Date of publication of application:
**26.02.86 Bulletin 86/09**

(45) Publication of the grant of the patent:
**30.08.89 Bulletin 89/35**

(84) Designated Contracting States:
**AT BE CH DE FR GB IT LI LU NL SE**

(56) References cited:
**EP-A-0 103 381**
**EP-A-0 114 002**
**DE-A-1 816 086**
**GB-A-1 431 617**

(73) Proprietor: **WARNER-LAMBERT COMPANY**
**201 Tabor Road**
**Morris Plains New Jersey 07950 (US)**

(72) Inventor: **Capps, David B.**
**1406 Brooklyn**
**Ann Arbor, MI 48104 (US)**
Inventor: **Werbel, Leslie M.**
**1570 Covington**
**Ann Arbor, MI 48103 (US)**
Inventor: **Showalter, Hollis H. D.**
**900 Patricia**
**Ann Arbor, MI 48103 (US)**

(74) Representative: **Jones, Michael Raymond et al**
**HASELTINE LAKE & CO. Hazlitt House 28**
**Southampton Buildings Chancery Lane**
**London WC2A 1AT (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to novel substituted benzopyrano[4,3,2-*cd*]indazoles, to methods for their production, to pharmaceutical compositions comprising the compounds, and to these compounds for therapeutic use in dosage form. The compounds of the invention have pharmacological properties and are useful antibacterial agents, antifungal agents, and antitumor agents.

The benzopyrano[4,3,2-*cd*]indazole ring system is new and is illustrated by the formula

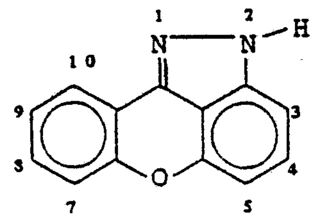

The corresponding benzothiopyrano[4,3,2-*cd*]indazoles, containing —S— instead of —O—, are disclosed in EP—A—0 114 002, which was published after the priority date of the present application but has an earlier priority date.

The present invention in one aspect relates to benzopyrano[4,3,2-*cd*]indazole compounds having, in free base form, the structural Formula *1*:

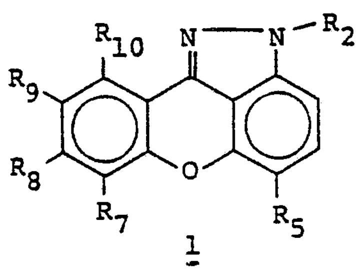

1

and to the pharmaceutically acceptable salts thereof, wherein the substituents $R_7$, $R_8$, $R_9$, and $R_{10}$ represent hydrogen, hydroxy or alkoxy of from one to four carbon atoms, hereinafter referred to as lower alkoxy; wherein $R_2$ is ANR'R'' wherein A is a straight or branched alkylene chain of from two to five carbon atoms, optionally substituted with hydroxyl; wherein R' and R'' are hydrogen or straight or branched alkyl of from one to four carbon atoms, optionally substituted with hydroxyl, R' and R'' taken together also representing

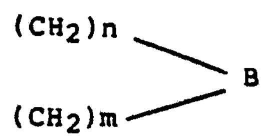

wherein n and m are each an integer from two to three and B is a direct bond or O, S, or NR''' wherein R''' is hydrogen or straight or branched alkyl of from one to four carbon atoms, optionally substituted with hydroxyl; and wherein $R_5$ is nitro, $NH_2$, $NHR_2$, or NHR'''' wherein R'''' is

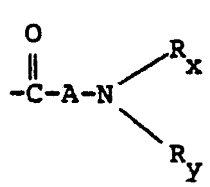

wherein A is straight or branched alkylene of from one to four carbon atoms, $R_x$ and $R_y$ are each hydrogen or straight or branched alkyl of from one to four carbon atoms optionally substituted by hydroxy or

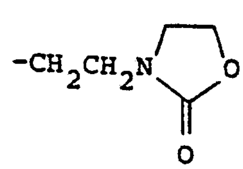

The compounds of the invention form pharmaceutically acceptable salts with both organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic, isethionic, lactic, gluconic, glucuronic, sulfamic, benzoic, tartaric, pamoic, and the like. The salts are prepared by contacting the free base form with an equivalent amount of the desired acid in the conventional manner. The free base forms may be regenerated by treating the salt form with a base. For example, dilute aqueous base solutions may be utilized. Dilute aqueous sodium hydroxide, potassium carbonate, ammonia, and sodium bicarbonate solutions are suitable for this purpose. The free base forms differ from their respective salt forms somewhat in certain physical properties such as solubility in polar solvents, but the salts are otherwise equivalent to their respective free base forms for purposes of the invention.

The compounds of the invention can exist in unsolvated as well as solvated forms, including hydrated forms. In general, the solvated forms with pharmaceutically acceptable solvents such as water, ethanol, and the like are equivalent to the unsolvated forms for purposes of the invention.

The term halogen as used herein is intended to include fluorine, chlorine, bromine, and iodine.

The invention in another aspect relates to benzopyrano[4,3,2-*cd*]indazole compounds having the structural Formula *2*:

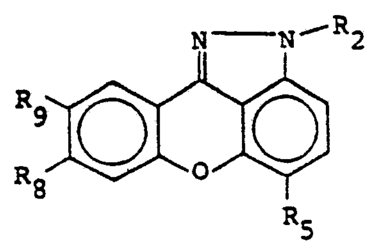

2

and the pharmaceutically acceptable salts thereof; wherein $R_2$, and $R_5$ are as defined above, and $R_8$ and $R_9$ are independently hydrogen, hydroxy, or lower alkoxy.

Preferred compounds of the present invention include those wherein $R_5$ is nitro, amino, or —$NHR_2$ where R' and R'' are hydrogen or straight or branched alkyl of from one to four carbon atoms optionally substituted with hydroxyl or R' and R'', when taken together with the nitrogen atom to which they are attached, form a ring represented by

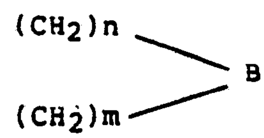

where n and m are each an integer of from two to three and B is a direct bond.

Other preferred compounds are those wherein $R_2$ is diethyl aminoethyl and $R_5$ is $NHCH_2CH_2NH_2$.

The invention in another aspect relates to compounds that are most preferred for their pharmacological properties, these compounds having the following names:

N-[2-[2-(diethylamino)ethyl]-2*H*-[1]-benzopyrano[4,3,2-*cd*]indazol-5-yl]ethanediamine and

N-[2-[2-(diethylamino)ethyl]-9-hydroxy-2*H*-[1]-benzopyrano[4,3,2-*cd*]indazol-5-yl]ethanediamine.

Further preferred are:

*N,N*-diethyl-9-methoxy-5-nitro-2*H*-[1]-benzopyrano[4,3,2-*cd*]indazole-2-ethanamine;

*N,N*-diethyl-9-hydroxy-5-nitro-2*H*-[1]benzopyrano[4,3,2-*cd*]indazole-2-ethanamine;

9-methoxy-*N,N*-dimethyl-5-nitro-2*H*-[1]benzopyrano[4,3,2-*cd*]indazole-2-propanamine;

2-[3-(dimethylamino)propyl]-5-nitro-2*H*-[1]benzopyrano[4,3,2-*cd*]indazol-9-ol;

5-amino-2-[2-(diethylamino)ethyl]-2*H*-[1]benzopyrano[4,3,2-*cd*]indazol-9-ol;

5-amino-2-[2-[(2-hydroxyethyl)amino]ethyl]-2*H*-[1]benzopyrano[4,3,2-*cd*]indazol-9-ol;

*N*-[2-[2-(diethylamino)ethyl]-9-hydroxy-2*H*-[1]benzopyrano[4,3,2-*cd*]indazol-5-yl]-2-[(2-hydroxyethyl)-amino]acetamide;

2-[2-[(2-hydroxyethyl)amino]ethyl]-5-nitro-2*H*-[1]benzopyrano[4,3,2-*cd*]indazol-9-ol;

2-[2-diethylamino)ethyl]-5-[[2-[(2-hydroxyethyl)amino]ethyl)amino-2*H*-[1]benzopyrano[4,3,2-*cd*]-indazol-9-ol;

2-[2-[(2-hydroxyethyl)amino]ethyl]-9-hydroxy-2*H*-[1]benzopyrano[4,3,2-*cd*]indazol-5-yl]-2-[(2-hydroxyethyl)amino]acetamide;

2-[2-[(2-hydroxyethyl)amino]ethyl]-5-[[2-[(2-hydroxyethyl)amino]ethyl]amino]-2*H*-[1]benzopyrano-[4,3,2-*cd*]indazol-9-ol;

9-methoxy-2-[2-(1-pyrrolidinyl)ethyl]-2*H*-[1]benzopyrano[4,3,2-*cd*]indazol-5-amine;

5-nitro-2-[2-(1-pyrrolidinyl)ethyl]-2*H*-[1]benzopyrano[4,3,2-*cd*]indazol-9-ol; and

5-[(2-aminoethyl)amino]-2-[2-(diethylamino)ethyl]-2*H*-[1]benzopyrano[4,3,2-*cd*]indazol-8-ol.

Certain of the compounds of the invention are also useful as intermediates in the preparation of the preferred species; more specifically, those compounds of structure *1* wherein $R_5$ represents $NO_2$ or $NH_2$ and those compounds such as those of structures 5, 6, and 7 described below which contain protecting groups.

The invention in another aspect also includes novel intermediates such as, for example, compounds of the formula:

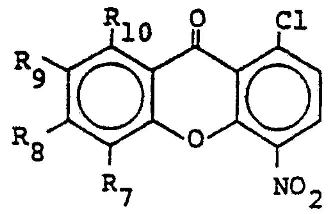

wherein $R_7$, $R_8$, $R_9$, and $R_{10}$ are each hydrogen, hydroxy or lower alkoxy.

Preferred of these intermediates are compounds of the formula:

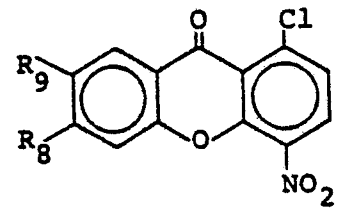

wherein $R_8$ and $R_9$ are each independently hydrogen, hydroxy or lower alkoxy.

Process for Preparing the Compounds

The invention in one process aspect comprises a process for preparing compounds having the structural formula:

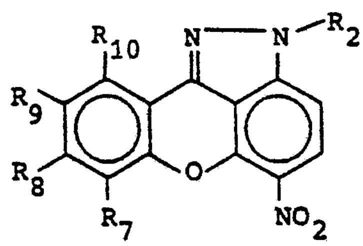

by reacting a suitably substituted 1-halo-4-nitro-9*H*-xanethen-9-one and $R_2$-substituted hydrazine having the structural formula $H_2NNHR_2$, wherein $R_2$, $R_7$, $R_8$, $R_9$, and $R_{10}$ have the above meaning. The reaction conditions can be varied widely. The reaction is usually carried out in a solvent at temperatures between about 25 to about 140°C. A suitable solvent is acetonitrile, 1,2-dichloroethane, tetrahydrofuran, methanol or DMF.

The requisite hydrazines are prepared by reaction of hydrazine with the appropriate alkyl halide, $XR_2$, wherein $R_2$ has the above meaning [*J. Med. Chem.*, *7*, 403 (1964)], or other methods known in the art as described below.

The invention in another aspect comprises a process for preparing compounds having the structural Formula *3*:

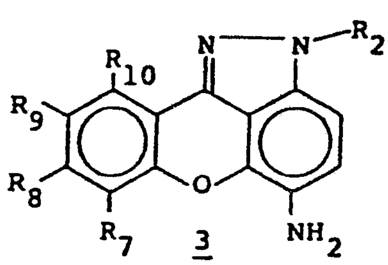

3

by subjecting a compound having the structural formula:

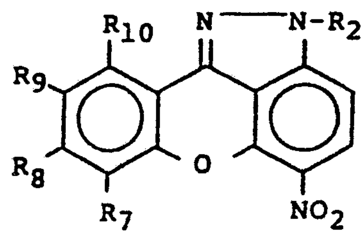

to reduction, wherein $R_2$, $R_7$, $R_8$, $R_9$, and $R_{10}$ have the above meaning. The reduction is carried out by suitable means, preferably by hydrogenation at room temperature using a palladium/charcoal catalyst in a solvent such as methanol or acetic acid, acetic acid being preferred.

The invention in another aspect comprises a process for preparing compounds having the structural formula *4*:

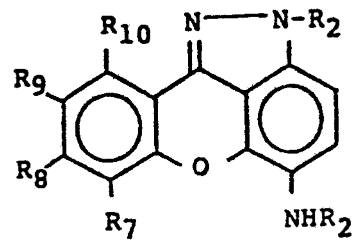

4

by reacting a compound having the structural formula *3*:

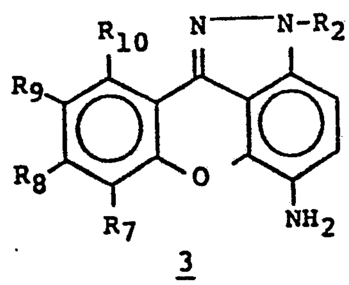

3

with a haloalkylamine having the formula $XR_2$, wherein $R_2$, $R_7$, $R_8$, $R_9$, and $R_{10}$ have the above meaning and X is a halogen. Where $R_2$ includes a sensitive group, this is appropriately protected prior to the reaction by a protective group. The reaction is carried out in the absence of solvent or in a suitable unreactive solvent such as $CHCl_3$ or DMF. In the absence of solvent, the reaction temperature may be about 150°C. With solvent, reflux temperature is used, for example, from about 60 to about 150°C. Bases such as $Et_3N$ or $K_2CO_3$ may be employed as acid scavengers but are not essential.

In another aspect, the invention comprises a process for preparing compounds having the structural Formula *4*:

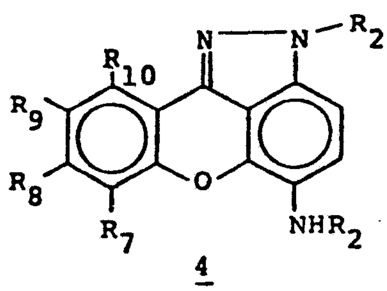

4

by reacting a compound having the Formula *3* above with the appropriately substituted aldehyde or acetal, ketone or ketal at reflux temperature, for example, with a haloalkylamine having the formula $XR_2$, wherein $R_2$, $R_7$, $R_8$, $R_9$, and $R_{10}$ have the above meaning and X is a halogen. Where $R_2$ includes a sensitive group, this

is appropriately protected prior to the reaction by a protective group. The reaction is carried out in the absence of solvent or in a suitable unreactive solvent such as $CHCl_3$ or DMF. In the absence of solvent, the reaction temperature may be about 150°C. With solvent, reflux temperature is used, for example, from about 60 to about 150°C. Bases such as $Et_3N$ or $K_2CO_3$ may be employed as acid scavengers but are not essential.

In another aspect, the invention comprises a process for preparing compounds having the structural Formula *4*:

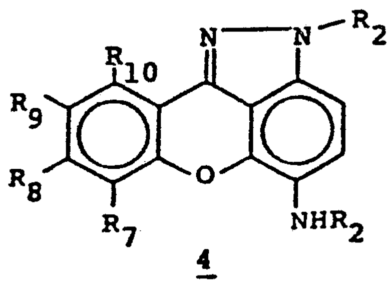

4

by reacting a compound having the Formula *3* above with the appropriately substituted aldehyde or acetal, ketone or ketal at reflux temperature, for example, from about 65 to about 140°C, and reducing the resulting Schiff base with a suitable reducing agent such as $NaBH_4$ or $NaBH_3CN$; wherein $R_2$, $R_7$, $R_8$, $R_9$, and $R_{10}$ have the above meaning.

In one preferred embodiment, the invention comprises a process for preparing benzopyrano[4,3,2-*cd*]indazole compounds having the structural Formula *1* above which comprises reacting an optionally substituted 1-chloro-4-nitro-9*H*-xanthen-9-one and an $R_2$ substituted hydrazine to form the compounds having the structural Formula *1* where $R_5$ is nitro and, if desired, converting said compounds by reduction to compounds having the structural Formula *1* where $R_5$ is $NH_2$ and if further desired converting said compounds to compounds having the structural Formula *1* where $R_5$ is $NHR_2$ by alkylation with a haloalkylamine having the formula $XR_2$, optionally after covering sensitive groups with protective groups; or by monoacylating with a reactive derivative of an acylating agent of corresponding to R'''' in which R'''' is defined above and reducing the acylated product, optionally after covering sensitive groups with protecting groups; and if desired, removing the protecting groups by hydrolysis or reduction; and isolating the product in free base or acid addition salt form; wherein X is chloro or bromo and $R_2$, R', $R_5$, $R_7$, $R_8$, $R_9$, and $R_{10}$ have the above meaning.

In another embodiment, the invention comprises a process for preparing benzopyrano[4,3,2-*cd*]indazole compounds having in free base form the structural formula:

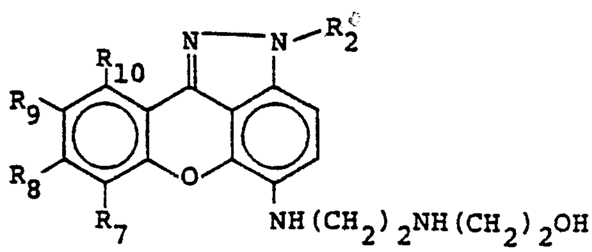

by reacting a compound having the structural formula:

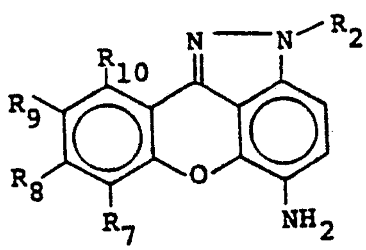

wherein $R_7$, $R_8$, $R_9$, and $R_{10}$ are hydrogen, hydroxyl, lower alkoxy, or benzyloxy, or *p*-halo or *p*-methoxy substituted benzyloxy, with 3-(β-haloethyl)-2-oxazolidinone to obtain an oxazolidinone compound having the structural formula:

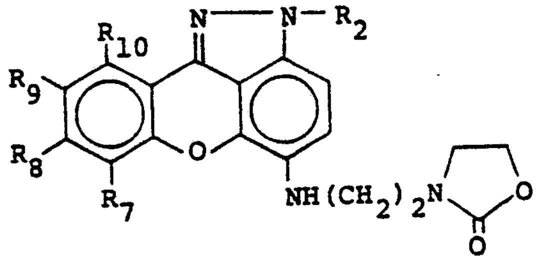

as defined above and subjecting the latter compound to alkaline hydrolysis to obtain a compound having the structural formula:

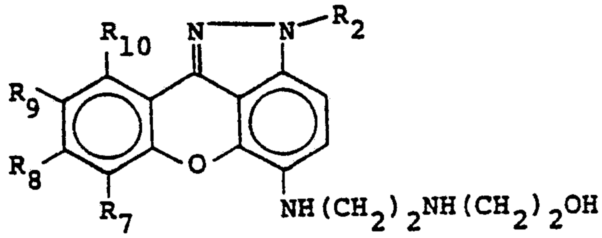

if necessary, removing any benzyloxy groups by hydrogenolysis, and isolating the product in free base or acid addition salt form. The invention also contemplates the embodiment in which one or more of $R_7$, $R_8$, $R_9$, and $R_{10}$ are benzyloxy or alkoxy wherein the benzyloxy and alkoxy groups are removed by hydrolysis or treatment with boron tribromide.

In still another embodiment, the invention comprises a process for preparing compound having the structural Formula *5*:

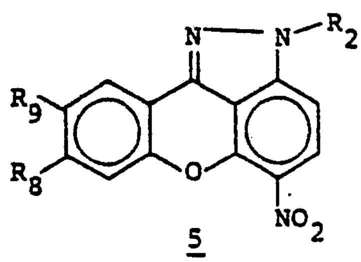

5

by reacting a compound having the structural formula:

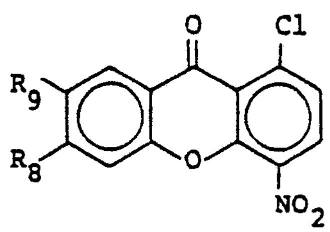

with a substituted hydrazine having the structural formula $H_2NNHR_2$; wherein $R_2$ has the above meaning and $R_8$ and $R_9$ are alkoxy of from one to four carbon atoms and may also be benzyloxy or halo- or methoxy-substituted benzyloxy.

In still another embodiment, the invention comprises a process for preparing compounds having the structural Formula *6*:

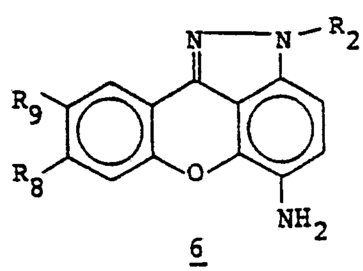

6

by subjecting to reduction a compound having the structural Formula *5*:

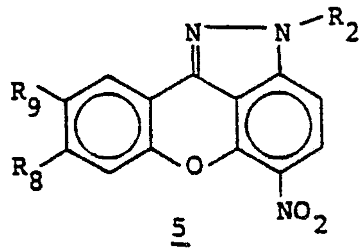

5

wherein $R_2$ has the above meaning and $R_8$ and $R_9$ are alkoxy of from 1 to 4 carbon atoms and may also be benzyloxy or halo- or methoxy-substituted benzyloxy. Preferably, the reduction is carried out in AcOH using Pd/C as the catalyst for the methoxy substituted compounds and in MeOH using Raney nickel as the catalyst for the benzyloxy substituted compounds.

The invention also comprises a process for preparing compounds having the structural Formula *7*:

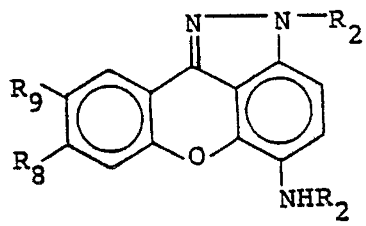

7

by alkylating a compound having the structural Formula *6*:

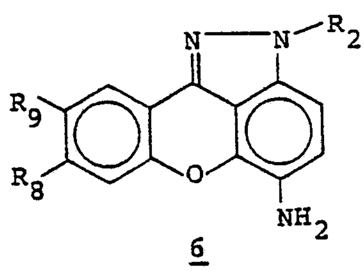

6

wherein $R_2$ has the above meaning and $R_8$ and $R_9$ are alkoxy of from 1 to 4 carbon atoms and may also be benzyloxy or *p*-halo- or *p*-methoxy-substituted benzyloxy.

The invention also provides a process for preparing a ocmpound having the following general formula:

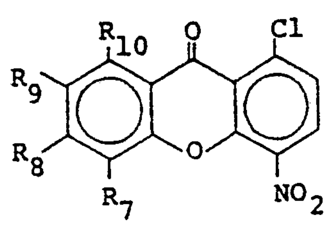

which comprises non-oxidatively dehydrating a compound having the formula:

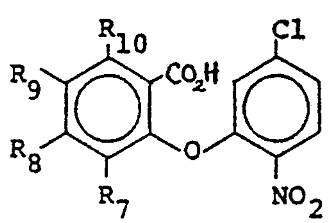

wherein $R_7$, $R_8$, $R_9$ and $R_{10}$ are as defined above.

The invention in its composition aspect relates to a pharmaceutical composition comprising a compound having structural Formula *1* and the pharmaceutically acceptable salts thereof in combination with a pharmaceutically acceptable carrier.

The invention in another aspect relates to a pharmaceutical composition comprising a compound having structural Formula *2* and the pharmaceutically acceptable salts thereof in combination with a pharmaceutically acceptable carrier.

The invention in another aspect relates to a pharmaceutical compositions comprising a compound having structural Formula *1* where $R_7$, $R_8$, $R_9$, and $R_{10}$ are hydrogen and the pharmaceutically acceptable salts thereof in combination with a pharmaceutically acceptable carrier.

The invention in another pharmaceutical aspect relates to a pharmaceutical composition comprising a compound having structural Formula *6* and the pharmaceutically acceptable salts thereof in combination with a pharmaceutically acceptable carrier.

The compounds of the invention can be used in a method for treating microbial infections in a mammal which comprises administering a sufficient amount of a compound having structural Formula *1* and the pharmaceutically acceptable salts thereof in combination with a pharmaceutically acceptable carrier, to a mammal in need thereof.

The compounds of the invention can be used in a method for treating leukemia in a mammal which comprises administering a sufficient amount of a compound having structural Formula *1* and the pharmaceutically acceptable salts thereof in combination with a pharmaceutically acceptable carrier, to a mammal in need thereof.

The compounds of the invention can be used in a method for treating leukemia in a mammal which comprises administering a sufficient amount of a compound having structural Formula *1* where $R_7$, $R_8$, $R_9$, and $R_{10}$ are hydrogen and the pharmaceutically acceptable salts thereof in combination with a pharmaceutically acceptable carrier, to a mammal in need thereof.

The compounds of the invention can be used in a method for treating solid tumors in a mammal which comprises administering a sufficient amount of a compound having structural Formula *1* and the pharmaceutically acceptable salts thereof in combination with a pharmaceutically acceptable carrier, to a mammal in need thereof.

The compounds of the invention can be used in a method for treating solid tumors in a mammal which comprises administering a sufficient amount of a compound having structural Formula *1* where $R_7$, $R_8$, $R_9$, and $R_{10}$ are hydrogen and the pharmaceutically acceptable salts thereof in combination with a pharmaceutically acceptable carrier, to a mammal in need thereof.

The compounds of the invention can be used in a method for treating solid tumors in a mammal which comprises administering a sufficient amount of a compound having structural Formula *1* where $R_7$, $R_8$, $R_9$, and $R_{10}$ are hydrogen and the pharmaceutically acceptable salts thereof in combination with a pharmaceutically acceptable carrier, to a mammal in need thereof.

The compounds of the invention can be used in a method for treating solid tumors in a mammal which comprises administering a sufficient amount of a compound having structural Formula *6* and the pharmaceutically acceptable salts thereof in combination with a pharmaceutically acceptable carrier, to a mammal in need thereof.

## Physical and Pharmacological Properties of the Compounds

The benzopyrano[4,3,2-*cd*]indazole compounds of the invention range in color from beige to yellow. They are generally crystalline solids that are stable under normal atmospheric conditions. The compounds typically have melting points in the range of about 100 to about 250°C.

The compounds are useful as pharmacological agents for the treatment of bacterial and fungal infections in warm-blooded animals. The activity of representative compounds of the invention was established by test protocols described below.

In addition to their usefulness as antibacterial and antifungal agents, compounds of the invention display in vitro and in vivo antitumor activity.

## Test Protocols

*In Vitro*

One test protocol is the in vitro proliferating human colon adenocarcinoma (HCA) cell screen. In this test, HCT-8 cells (HCA cell line received from Yale University) are trypsinized using trypsin-EDTA. A single cell suspension is achieved by passing the cells through a 26 gauge needle with a 20 cc syringe. A cell suspension is prepared using RPMI 1640 + 10% FCS + 50 µg/ml gentamicin sulfate with a cell concentration of approximately 30,000 cells/ml. The cell suspension is dispensed in Linbro 24-well plates; 1 ml/well. The plates are incubated for approximately 48 hours at 37°C in a 5% $CO_2$ atmosphere. At this time test compounds are added in the appropriate concentration. Five µl of the 200 µg/ml stock solution is added to each well in a primary test. Ten µl of the appropriate dilution is added to each well for the titration test. The plates are reincubated an additional 60 to 65 hours at 37°C in a 5% $CO_2$ atmosphere. The cells are lysed using a mix of cationic surfactant, glacial acetic acid, and sodium chloride. Two ml of the lysed cell suspension from each well is added to 8 ml of diluent. The number of nuclei is determined using a Coulter

counter (ZBI model), and a percent growth for each drug concentration is calculated. From this, an $ID_{50}$ (molar concentration of compound that results in 50% inhibition of growth) is determined.

Another test protocol uses L1210 cells, a murine leukemia cell line, grown in RPMI 1640 supplemented with 5% fetal bovine serum and gentamicin (50 µg/ml). Drug dilutions are prepared in the appropriate solvent and 20 µl of each dilution are added to 24-well Linbro tissue culture plates, followed by the addition of 2.0 ml of cell suspension containing $3 \times 10^4$ cells per ml. Solvent and medium controls are included in each test. After incubation at 37°C for three days in 5% $CO_2$, the contents of each well are removed and the cells counted in a ZBI Counter counter. Percent growth are calculated relative to the controls and the levels of drug activity are expressed as $ID_{50}$ in moles per liter.

Still another test protocol is the in vitro antibacterial/antifungal (ABF) test. Compounds are tested for antimicrobial activity in an agar-disk diffusion assay, a standard microbiological technique for testing antibiotics. After incubation of each culture with a test compound, a zone of inhibition is determined. The zone diameter (mm) of active compounds ranges from a minimum of 13.5 mm to as high as 60 mm, with a greater diameter reflecting higher activity. For convenience, values are reported for two gram-negative bacteria (*Aerobacter aerogenes* 0126 and *Escherichia coli* 04863), two gram-positive bacteria (*Bacillus subtilis* 04555 and *Streptococcus faecalis* 05045 utilizing AM-09 medium), and one mycelial fungus (*Penicillium avellaneum* M2988).

Still another test protocol is the in vitro antibacterial (ABMF) test which is a recognized standard microdilution susceptibility procedure in Mueller-Hinton broth against Gram-positive and Gram-negative bacterial test organisms. The procedure is a modification of a state-of-the-art procedure reported in *Manual of Clinical Microbiology*, Lennette, E. H., ed., by Barry, A. L. and C. Thornsberry at pages 463—474 and by Gavan, T. L. and A. L. Barry at pages 459—462, American Society for Microbiology, Washington, 1980.

In the test, a given bacterial culture is used to inoculate individual test wells of microdilution trays containing growth medium and test compound, the latter in a microdilution series: 1000, 333, 111, 37, 12, 4, 1.4, and 0.46 micrograms per milliliter. The resulting inoculated trays are each sealed, incubated with blank controls at 37°C for 16—24 hours, and then read for minimum inhibitory concentration (MIC), the lowest concentration of test compound that completely inhibits bacterial growth. MIC values lower than 333 mcg/ml indicate antimicrobial activity. For convenience, values are reported for *Escherichia coli*, *Corynebacterium* Sp. ATCC 21698, *Salmonella typhimurium*, *Streptococcus pneumoniae*, and *Rhodotorula aurantiaca.*

*In Vivo*

Another test protocol is the in vivo lymphocytic leukemia P388 test. The animals used are either male or female $CD_2F_1$ mice. There are six or seven animals per test group. The tumor transplant is by intraperitoneal injection of dilute ascitic fluid containing cells of lymphocytic leukemia P388. The test compounds are administered intraperitoneally once daily for five consecutive days at various doses following tumor inoculation. The animals are weighed and survivors are recorded on a regular basis for 30 days. A compound is designated "toxic" if, at a given dose, all animals died prior to four days after the first injection of drug. A ratio of survival time for treated (T)/control (C) animals is calculated. A criterion for efficacy is a ratio T/C times 100 greater than or equal to 125. See *Cancer Chemotherapy Reports*, Part 3, *3*, 1 (1972) for a comprehensive discussion of the protocol.

These test protocol procedures gave results listed in Tables 1, 2, and 3 for representative compounds of the present invention.

TABLE 1
Antitumor, Antibacterial, and Antifungal Data on
2,5-(Disubstituted)-2H-[1]benzopyrano[4,3,2-*cd*]indazoles

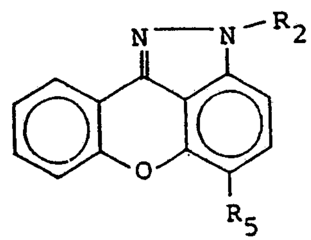

| $R_2$ | $R_5$ | L1210 in vitro $ID_{50}$ (M) | HCA-4 in vitro $ID_{50}$ (M) | P388 in vivo Dose (mg/kg) | P388 in vivo T/C×100 | ABF Zone Diameter (Conc. in mg/ml) A. Aero-genes | E. Coli | B. Sub-tilis | S. Fae-calis | P. Avell-aneum |
|---|---|---|---|---|---|---|---|---|---|---|
| $CH_2CH_2NEt_2$ | $NO_2$ | $1.9 \times 10^{-6}$ | $1 \times 10^{-6}$ | 200<br>100 | 211<br>138 | — | — | 14 (.5) | 15 (.5) | 0 (3) |
| $CH_2CH_2NHCH_2CH_2OH$ | $NO_2$ | $4 \times 10^{-7}$ | $5 \times 10^{-7}$ | 50 | 141 | — | — | 17 (.1) | 17 (.5) | 19 (.1) |
| $CH_2CH_2NEt_2$ | $NH_2$ | $1.3 \times 10^{-6}$ | $2.4 \times 10^{-6}$ | | | | | | | |
| $CH_2CH_2NEt_2$ | $NHCH_2CH_2NH_2$ | $8 \times 10^{-8}$ | $1.6 \times 10^{-7}$ | 25<br>12.5 | 216<br>174 | — | — | 14 (.5) | 16 (3) | 0 (3) |

TABLE 2

Antitumor, Antibacterial, and Antifungal Data on 2,5,9-Trisubstituted-2H-[1]benzopyrano[4,3,2-*cd*]indazoles

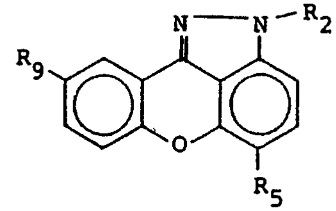

| PD Number (Example) | $R_9$ | $R_2$ | $R_5$ | L1210 in vitro $ID_{50}$ (M) | P388 in vivo Dose (mg/kg) | P388 in vivo T/C × 100 | ABMF Minimal Inhibitory Concentration (µ/ml) E. coli | S. typh. | Coryn. sp. | S. pneum. | R. aurant |
|---|---|---|---|---|---|---|---|---|---|---|---|
| 119,154 (5) | $CH_3O$ | $CH_2CH_2NEt_2$ | $NO_2$ | $9.2 \times 10^{-7}$ | 100<br>50<br>25 | 192<br>148<br>126 | <0.46 | 111.0 | <0.46 | <0.46 | 111.0 |
| 119,302 (6) | $CH_3O$ | $CH_2CH_2NEt_2$ | $NHCH_2CH_2NH_2$ | $1.1 \times 10^{-6}$ | 25<br>12.5 | 145<br>154 | 111.0 | 111.0 | 4.1 | 12.3 | 333.0 |
| 119,298 (7) | $CH_3O$ | $CH_2CH_2CH_2NMe_2$ | $NO_2$ | $5.8 \times 10^{-7}$ | 200<br>100<br>50 | 218<br>159<br>134 | 4.1 | 111.0 | <0.46 | <0.46 | 1000 |
| 122,143 (8) | $CH_3O$ | $CH_2CH_2N(CH_2)_4$ | $NO_2$ | $2.0 \times 10^{-6}$ | | | >1000 | 37.0 | <0.46 | <0.46 | 111.0 |
| 122,390 (9) | $CH_3O$ | $CH_2CH_2N(CH_2)_4$ | $NH_2$ | $3.6 \times 10^{-7}$ | | | 12.3 | 111.0 | 4.1 | 4.1 | 333 |
| 122,210 (10) | HO | $CH_2CH_2N(CH_2)_4$ | $NO_2$ | $3.5 \times 10^{-9}$ | | | >1000 | 1.4 | <0.46 | <0.46 | 1000 |
| 119,375 (11) | HO | $CH_2CH_2CH_2NMe_2$ | $NO_2$ | $1.5 \times 10^{-8}$ | 25<br>12.5<br>6.25 | 226<br>200<br>150 | <0.46 | 12.3 | <0.46 | <0.46 | 1000 |
| 119,454 (12) | HO | $CH_2CH_2NEt_2$ | $NH_2$ | $7.1 \times 10^{-8}$ | 25<br>12.5<br>6.25 | 192<br>201<br>167 | 111.0 | 111.0 | 12.3 | 12.3 | >1000 |

TABLE 2 (Cont'd)
Antitumor, Antibacterial, and Antifungal Data on
2,5,9-Trisubstituted-2H-[1]benzopyrano[4,3,2-*cd*]indazoles

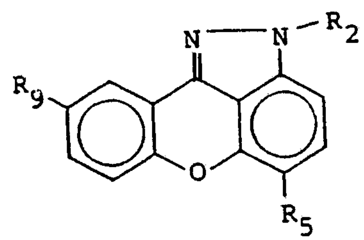

| PD Number (Example) | $R_9$ | $R_2$ | $R_5$ | L1210 in vitro $ID_{50}$ (M) | P388 in vivo Dose (mg/kg) | P388 in vivo T/C × 100 | ABMF Minimal Inhibitory Concentration (μ/ml) E. coli | S. typh. | Coryn. sp. | S. pneu. | R. aurant |
|---|---|---|---|---|---|---|---|---|---|---|---|
| 120,317 (13) | HO | $CH_2CH_2NEt_2$ | $NHC(O)CH_2NHCH_2CH_2OH$ | $5.7 \times 10^{-9}$ | 100<br>50<br>25 | 272<br>227<br>198 | 111.0 | >1000 | 333.0 | 333.0 | >1000 |
| 120,586 (14) | HO | $CH_2CH_2NEt_2$ | $NHCH_2CH_2NHCH_2CH_2OH$ | $4.8 \times 10^{-8}$ | 12.5<br>6.25 | 272<br>236 | 11.0 | >33.0 | 33.0 | 11.0 | >33.0 |
| 120,582 (15) | HO | $CH_2CH_2NHCH_2CH_2OH$ | $NO_2$ | $1.5 \times 10^{-8}$ | 50<br>25<br>12.5 | 230<br>218<br>190 | >0.46 | 12.3 | 1.4 | 1.4 | 1000 |
| 120,072 (16) | HO | $CH_2CH_2NHCH_2CH_2OH$ | $NH_2$ | $1.2 \times 10^{-7}$ | 25<br>12.5<br>6.25 | 260<br>200<br>165 | 4.1 | 37.0 | 12.3 | 37.0 | >1000 |
| 120,853 (17) | HO | $CH_2CH_2NHCH_2CH_2OH$ | $NHC(O)CH_2NHCH_2CH_2OH$ | $1.7 \times 10^{-6}$ | 240<br>120<br>60 | 184<br>142<br>134 | 1000 | >1000 | 333.0 | >1000 | 1000 |
| 121,166 (18) | HO | $CH_2CH_2NHCH_2CH_2OH$ | $NHCH_2CH_2NHCH_2CH_2OH$ | $1.3 \times 10^{-7}$ | 25<br>12.5<br>6.25 | 236<br>236<br>173 | 111.0 | 1000 | 37.0 | 333.0 | >1000 |

TABLE 3
Antitumor, Antibacterial, and Antifungal Data on
2,5,8-Trisubstituted-2H-[1]benzopyrano[4,3,2-*cd*]indazoles

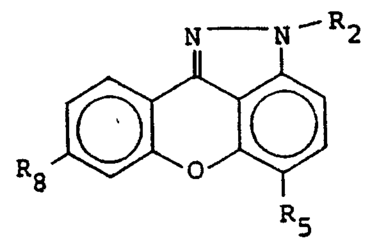

| PD Number (Example) | $R_8$ | $R_2$ | $R_5$ | L1210 in vitro $ID_{50}$ (M) | ABMF Minimal Inhibitory Concentration (μ/ml) E. coli | S. typh. | Coryn. bact. sp. | S. pneum. | R. aurant. |
|---|---|---|---|---|---|---|---|---|---|
| 122,760 (19) | $CH_3O$ | $CH_2CH_2N(Et)_2$ | $NO_2$ | $2.2 \times 10^{-6}$ | <0.46 | 37 | <0.46 | <0.46 | 333 |
| 122,757 (20) | HO | $CH_2CH_2N(Et)_2$ | $NO_2$ | $8.7 \times 10^{-7}$ | 1.4 | 12.3 | <0.46 | <0.46 | >1000 |

## Preparation of Pharmaceutical Compositions

When being utilized as antibiotic and antifungal agents, the compounds of the invention can be prepared and administered in a wide variety of topical, oral, and parenteral dosage forms. It will be clear to those skilled in the art that the following dosage forms may comprise as the active component, one or more compounds of Formula *1*, a corresponding pharmaceutically acceptable salt of any of said compounds, or a mixture of such compounds and/or salts.

For preparing pharmaceutical compositions from the compounds described by this invention, inert, pharmaceutically acceptable carriers can be either solid or liquid. Solid form preparations include powders, tablets, dispersible granules, capsules, cachets, and suppositories. A solid carrier can be one or more substances which may also act as diluents, flavoring agents, solubilizers, lubricants, suspending agents, binders, or tablet disintegrating agents; it can also be an encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 or 10 to about 70 percent of the active ingredient. Suitable solid carriers are magnesium carbonate, magnesium stearate, talc, sugar, lactose, pectin, dextrin, starch, gelatin, tragacanth, methyl cellulose, sodium carboxymethyl cellulose, a low melting wax, cocoa butter, and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets, and capsules can be used as solid dosage forms suitable for oral administration.

Liquid form preparations include solutions, suspensions, and emulsions. As an example may be mentioned water or water-propylene glycol solutions for parenteral injection. Liquid preparations can also be formulated in solution in aqueous polyethylene glycol solution. Aqueous solutions suitable for oral use can be prepared by dissolving the active component in water and adding suitable colorants, flavors, stabilizing, and thickening agents as desired. Aqueous suspensions suitable for oral use can be made by dispersing the finely divided active component in water with viscous material, i.e., natural or synthetic gums, resins, methyl cellulose, sodium carboxymethyl cellulose, and other well-known suspending agents.

Topical preparations included dusting powders, creams, lotions, gels, and sprays. These various topical preparations may be formulated by well-known procedures. See for example Remington's Pharmaceutical Sciences, Chapter 43, 14th Ed., 1970, Mack Publishing Co., Easton, Pennsylvania 18042, USA.

Preferably, the pharmaceutical preparation is in unit dosage form. In such form, the preparation is subdivided into unit doses containing appropriate quantities of the active component. The unit dosage form can be packaged preparation, the package containing discrete quantities of preparation, for example, packeted tablets, capsules, and powders in vials or ampoules. The unit dosage form can also be a capsule, cachet, or tablet itself or it can be the appropriate number of any of these packaged forms.

The quantity of active compound in a unit dose of preparation may be varied or adjusted from 50 mg to 500 mg according to the particular application and the potency of the active ingredient.

In therapeutic use as antibiotic and antifungal agents, the compounds utilized in the pharmaceutical method of this invention are administered at the initial dosage of about 0.1 mg to about 50 mg per kilogram. A dose range of about 0.5 mg to about 10 mg per kilogram is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The active compounds may also be administered parenterally or intraperitoneally. Solutions of the active compound as a free base or pharmaceutically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like, N,N-dimethylacetamide, suitable mixtures thereof and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the

injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by sterilization accomplished by filtering. Generally, dispersions are prepared by incorporating the various sterilized active ingredient into a sterile vehicle which contains the basic dispersion medium and the required other ingredients from those enumerated above. In the case of the sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well-known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in unit dosage form for ease of administration and uniformity of dosage. Unit dosage form as used herein refers to physically discrete units suitable as unitary dosages for the mammalian subjects to be treated; each unit containing a predetermined quantity of active material calculated to produce the desired therapeutic effect in association with the required pharmaceutical carrier. The specification for the novel unit dosage forms of the invention are dictated by and directly dependent on (a) the unique characteristics of the active material and the particular therapeutic effect to be achieved, and (b) the limitation inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in unit dosage form as hereinbefore disclosed. A unit dosage form can, for example, contain the principal active compound in amounts ranging from about 0.1 to about 500 mg, with from about 0.5 to about 250 mg being preferred. Expressed in proportions, the active compound is generally present in from about 0.1 to about 500 mg/ml of carrier. In the case of compositions containing supplementary active ingredients, the dosages are determined by reference to the usual dose and the manner of administration of the said ingredients. The daily parenteral doses for mammalian subjects to be treated ranges from 0.1 mg/kg to 100 mg/kg. The preferred daily dosage range is 0.3 mg/kg to 10 mg/kg.

The invention and the best mode of practicing the same are illustrated by the following examples of preferred embodiments of selected compounds and their preparation.

Example 1

*N,N*-Diethyl-5-nitro-2*H*[1]benzopyrano[4,3,2-*cd*]indazole-2-ethanamine

A solution of 0.83 g of crude 1-chloro-4-nitro-9*H*-xanthen-9-one and 1.0 g of [2-(diethylamino)ethyl]-hydrazine in 15 ml of tetrahydrofuran was stirred at room temperature for four hours. The mixture was evaporated to dryness and the residue was triturated with water, collected, and recrystallized from ethyl acetate to provide the product mp 158—160°C. The methanesulfonic acid salt mp 179—180°C crystallized from a solution of the base in a mixture of methanol and diethylether containing an equivalent of methanesulfonic acid.

The 1-chloro-4-nitro-9*H*-xanthen-9-one was prepared as follows: Crude 2-(5'-chloro-2-nitrophenoxy)-benzoic acid (50 g) was added to 200 g of concentrated sulfuric acid. The mixture was held at 75°C for 4.5 hours, and added to a stirred mixture of 1.5 l of chloroform and water containing ice as needed to maintain the temperature below 35°C. The chloroform layer was separated, washed with 5% sodium bicarbonate, evaporated to dryness and the residue was crystallized from 200 ml of toluene and 30 ml of cyclohexane to provide the product mp 175—186°C.

The 2-(5'-chloro-2-nitrophenoxy)benzoic acid was prepared as follows: Crude 2-(5-chloro-2-nitrophenoxy)benzaldehyde was heated under reflux with 200 ml of diethylether, 120 ml of water, 41.1 g of $Na_2Cr_2O_7 \cdot 2H_2O$ and 6.0 g of a 40% tetra-*n*-butylammonium hydroxide solution, and to it was added 63 ml of 9M $H_2SO_4$ over three hours. Heating was continued for an additional two hours, and the aqueous layer was extracted with ether and evaporated to dryness to provide the product.

The 2-(5-chloro-2-nitrophenoxy)benzaldehyde was prepared as follows: To 16.2 g of 2,4-dichloronitrobenzene stirred at 170°C was added 7.1 g of the potassium salt of salicylaldehyde, monohydrate, in portions over 1.5 hours. The mixture was stirred at 170°C for two hours, triturated with 150 ml of $CH_2Cl_2$ and filtered. The filtrate was evaporated to a red oil and the excess dichloronitrobenzene was removed by steam distillation to leave the product.

Example 2

2-[[2-(5-Nitro-2*H*-[1]benzopyrano[4,3,2-*cd*]-indazol-2-yl)ethyl]amino]ethanol, monohydrochloride

A suspension of 2.76 g of 1-chloro-4-nitro-9*H*-xanthen-9-one, 30 ml of THF, 15 ml of methanol and 1.40 g of 2-[(hydrazinoethyl)amino]-ethanol was stirred at room temperature for three days. The precipitate was collected, washed with THF and dried, providing the title compound, mp 262—64°C (decomp.).

Example 3

5-Amino-*N,N*-diethyl-2*H*-[1]benzopyrano[4,3,2-*cd*]indazol-2-ethanamine

A solution of 8.9 g (0.026 mole) of *N,N*-diethyl-5-nitro-2*H*-[1]benzopyrano[4,3,2-*cd*]indazole-2-ethanamine in 100 ml of tetrahydrofuran was hydrogenated over 1 g of 10% palladium on carbon at ambient temperature and a hydrogen pressure of 319000 to 361000 N/m$^2$ (46.3—52.3 psi). The mixture was filtered and the solvent removed in vacuo. The oily residue was triturated with petroleum ether and recrystallized from *n*-hexane to give the product, mp 78—80°C.

Example 4

N-[2-[2-(Diethylamino)ethyl]-2*H*-[1]benzopyrano[4,3,2-*cd*]indazol-5-yl]ethanediamine, trihydrobromide, dihydrate

A solution of 2.47 g of *N,N*-diethyl-5-nitro-2*H*-[1]benzopyrano[4,3,2-*cd*]indazole-2-ethanamine in 45 ml of THF was hydrogenated in the presence of 10% palladium on carbon hydrogenation catalyst to 5-amino-*N,N*-diethyl-2*H*-[1]benzopyrano[4,3,2-*cd*]indazol-2-ethanamine. The catalyst was removed, the solvent replaced with 35 ml of absolute ethanol, 4.4 g of 2-bromoethylamine hydrobromide added, and the mixture heated three days under reflux. The mixture was filtered, evaporated to dryness, and the residue crystallized from ethanol, providing the title compound, mp 235—48°C (decomp.).

Example 5

N,N-Diethyl-9-methoxy-5-nitro-2*H*-[1]benzopyrano-[4,3,2-*cd*]indazole-2-ethanamine, methanesulfonate (1:1)

A mixture of 25 g of 1-chloro-7-methoxy-4-nitro-9*H*-xanthen-9-one, and 21.48 g of [2-(diethylamino)ethyl]hydrazine in 500 ml DMF was stirred at room temperature for 45 minutes. The mixture was evaporated to dryness, and the residue was triturated with isopropyl alcohol and the yellow crystals collected as the title compound free base, mp 162—165°C.

The title salt, mp 227—229°C, crystallized from a methanol solution of the base and an equivalent of methanesulfonic acid.

Example 6

1-[2-[2-(Diethylamino)ethyl]-9-methoxy-2*H*-[1]benzopyrano-[4,3,2-*cd*]indazole-5-yl]-1,2-ethanediamine, trihydrobromide

A mixture of 5 g of *N,N*-diethyl-9-methoxy-5-nitro-2*H*-[1]benzopyrano[4,3,2-*cd*]indazole-2-ethanamine, and 0.940 g of 10% palladium on carbon, in 250 ml of tetrahydrofuran was stirred under hydrogen atmosphere for 18 hours, filtered, and the filtrate concentrated to give 5-amino-*N,N*-diethyl-9-methoxy-2*H*-[1]benzopyrano[4,3,2-*cd*]indazole-2-ethanamine.

A mixture of 4.9 g of 5-amino-*N,N*-diethyl-9-methoxy-2*H*-[1]benzopyrano[4,3,2-*cd*]indazole-2-ethanamine and 8.7 g of bromoethylamine hydrobromide in 150 ml of ethyl alcohol was heated under reflux for 100 hours, cooled to room temperature and filtered affording the title compound, mp 272—275°C.

Example 7

9-Methoxy-*N,N*-dimethyl-5-nitro-2*H*-[1]benzopyrano[4,3,2-*cd*]indazole-2-propanamine, methanesulfonate (1:1.17)

A mixture of 2 g of 1-chloro-7-methoxy-4-nitro-9*H*-xanthen-9-one and 1.53 g of [3-(dimethylamino)-propyl]hydrazine in 40 ml of D.M.F. was stirred at room temperature for 16 hours, and then evaporated to dryness. The residue was triturated with isopropyl alcohol, and the yellow solid collected as the title compound free base.

The title salt, mp 119—121°C, crystallized from a methanol solution of the base and 1.25 equivalents of methanesulfonic acid.

Example 8

9-Methoxy-5-nitro-2-[2-(1-pyrrolidinyl)ethyl]-2*H*-[1]benzopyrano[4,3,2-*cd*]indazole, monohydrochloride

A mixture of 12.2 g of 1-chloro-7-methoxy-4-nitro-9*H*-xanthen-9-one and 11.0 g of 1-(2-hydrazinoethyl)pyrrolidine in 200 ml of THF was stirred 20 hours at room temperature. The orange liquor was decanted, freed of solvent under reduced pressure, and the residue, in chloroform, was chromatographed over silica gel, eluting with chloroform-methanol (25:1 by volume). The desired fractions yielded the free base of the title compound, mp 140—142°C.

The hydrochloride salt, mp 251—254°C (decomp.) crystallized from a solution of the base in aqueous methanolic hydrochloric acid upon addition of ethyl acetate.

Example 9

9-Methoxy-2-[2-(1-pyrrolidinyl)ethyl]-2*H*-[1]benzopyrano[4,3,2-*cd*]indazole-2-amine, monohydrochloride

In 50 ml of THF 3.8 g of 9-methoxy-5-nitro-2-[2-(1-pyrroldinyl)ethyl]-2*H*-[1]benzopyrano[4,3,2-cd]-indazole was hydrogenated at atmospheric pressure and room temperature in the presence of 1.0 g of 10% palladium on carbon hydrogenation catalyst over a period of four hours. The mixture was filtered, the filtrate freed of solvent under reduced pressure and the residue converted to the title salt, mp 218—221°C (decomp.) in ethyl acetate containing an equivalent of 1N methanolic hydrogen chloride.

Example 10

5-Nitro-2[2-(1-pyrrolidinyl)ethyl]-2*H*-[1]benzopyrano[4,3,2-*cd*]indazole-9-ol, methanesulfonate

A solution of 10 g of 1-chloro-7-hydroxy-4-nitro-9*H*-xanthen-9-one and 9 g of 1-(2-hydrazinoethyl)-pyrrolidine in 200 ml of *N,N*-dimethylformamide was stirred 18 h at room temperature and then the solvent was removed in vacuo. The residue was triturated in 450 ml of 2-propanol to provide 10.32 g of the free base, mp 225—229°C. A slurry of 2.5 g of the base in 100 ml of methanol was treated with 0.5 ml of methanesulfonic acid. After a few drops of water was added to effect solution, the mixture was filtered over Celite® and the filtrate was diluted with 100 ml of ethyl acetate. Upon concentrating the volume in vacuo to 100 ml, yellow crystals formed which were collected by filtration and washed with ethanol to provide the title salt, mp 251—252°C.

Example 11

9-Hydroxy-*N,N*-dimethyl-5-nitro-2*H*-[1]benzopyrano[4,3,2-*cd*]indazole-2-propanamine, methanesulfonate (1:1)

A mixture of 2 g of 1-chloro-7-hydroxy-4-nitro-9*H*-xanthen-9-one and 1.61 g of [3-(dimethylamino)-propyl]hydrazine in 40 ml of D.M.F. was stirred for ten minutes at room temperature and then evaporated to dryness. The solid residue was triturated with isopropyl alcohol, and the yellow crystals collected to afford the title compound as the free base.

The title salt, mp 269—272°C (decomp.), crystallized from a methanol solution of the base and an equivalent of methanesulfonic acid.

Example 12

5-Amino-*N,N*-diethyl-9-hydroxy-2*H*-[1]benzopyrano[4,3,2-*cd*]indazole-2-ethanamine, dihydrochloride

A mixture of 1.25 g of 1-chloro-7-hydroxy-4-nitro-9*H*-xanthen-9-one, and 1.12 g of [2-(diethylamino)-ethyl]hydrazine in 25 ml of DMF was stirred at room temperature for 45 minutes, and then evaporated to dryness. The residue was triturated with isopropyl alcohol providing yellow crystals of *N,N*-diethyl-9-hydroxy-5-nitro-2*H*-[1]benzopyrano[4,3,2-*cd*]indazole-2-ethanamine.

A mixture of *N,N*-diethyl-9-hydroxy-5-nitro-2*H*-[1]benzopyrano[4,3,2-*cd*]indazole-2-ethanamine, and 450 mg of 20% palladium on carbon, in 250 ml of tetrahydrofuran was stirred under a hydrogen atmosphere for 18 hours, filtered, and the filtrate concentrated in vacuo to give the title compound as the free base.

The title salt, crystallized from a 2-propanol solution of the base and two equivalents of hydrochloric acid, mp >300°C.

Example 13

*N*-[2-[2-(Diethylamino)ethyl]-9-hydroxy-2*H*-[1]benzopyrano[4,3,2-*cd*]indazole-5-yl]-2-[(2-hydroxyethyl)-amino]acetamide, hydrochloride

To a soltuion of 0.46 g of 2-[2-(diethylamino)-ethyl]-5-amino-2H-[1]benzopyrano[4,3,2-*cd*]indazol-9-ol in 25 ml N,N-dimethylformamide was added 0.31 g of *N'*-(ethyl carbonimidoyl)-N,N-dimethyl-1,3-propanediamine monohydrochloride, and 1.03 g of N-[2-(phenylmethoxy)ethyl]-*N*-(phenylmethyl)glycine.

After 1.5 hours, the reaction was poured into 20 ml of aqueous saturated sodium bicarbonate solution and extracted with dichloromethane. The extracts were dried and concentrated in vacuo. The resulting solid was chromatographed on silica gel with a solution of 5% methanol in dichloromethane as eluent to afford *N*-[2-[2-(diethylamino)ethyl]-9-hydroxy-2*H*-[1]benzopyrano[4,3,2-*cd*]indazol-5-yl]-2-[[2-(phenyl-methoxy)-ethyl] (phenylmethyl)amino]acetamide, mp 165—168°C.

A mixture of 1.3 g of the above acetamide and 0.10 g of 20% palladium hydroxide on carbon in 50 ml of glacial acetic acid stirred under hydrogen at atmospheric pressure until the hydrogen uptake measured 100 ml. The raction mixture was filtered and coevaporated several times with methanol. The residue was dissolved in methanol and acidified with a solution of hydrogen chloride in 2-propanol. Further dilution with 2-propanol and cooling caused precipitation of a crystalline solid. The solid was isolated and dried to give 0.976 g of the product as a yellow crystalline solid with 2.5 equivalents of hydrogen chloride and 1.5 equivalents of water, mp 285°C with decomposition.

Example 14

2-[2-(Diethylamino)ethyl]-5-[[2-[(2-hydroxyethyl)amino]ethyl]amino-2*H*-[1]benzopyrano[4,3,2-*cd*]indazol-9-ol, hydrochloride (1:3.5)

To a mixture of 2.45 g of *N*-[2-[2-(diethylamino)ethyl]-9-hydroxy-2*H*-[1]benzopyrano[4,3,2-*cd*]indazol-5-

yl]-2-[[2-(phenylmethoxy)ethyl] (phenylmethyl)amino]acetamide in 80 mol of dry tetrahydrofuran under argon was added 40 ml of 1M solution of borane tetrahydrofuran complex in tetrahydrofuran. After 24 hours at 50°C, an additional 3 ml of borane tetrahydrofuran complex was added and the reaction continued for another three hours at 50°C. The mixture was poured into 150 ml of methanol, heated under reflux overnight, evaporated to dryness, and the residue of crystallized from diethyl ether to afford 1.36 g of 2 - [2 - (diethylamino)ethyl] - 5 - [[2 - [[2 - (phenylmethoxy)ethyl] (phenylmethyl)amino]ethyl]amino) - 2*H* - [1]benzopyrano[4,3,2 - *cd*]indazol - 9 - ol.

A mixture of 1.3 g of the above material was debenzylated in 50 ml of glacial acetic acid in an atmosphere of hydrogen at atmospheric pressure in the presence of 0.2 g of 20% palladium on carbon over a period of 48 hours. The mixture was filtered, concentrated under reduced pressure, and reevaporated several times from methanol. The residue in 2-propanol was acidified with hydrogen chloride in 2-propanol, providing the title compound.

Example 15

2-[2-[(2-Hydroxyethyl)amino]ethyl]-5-nitro-2*H*-[1]benzopyrano[4,3,2-*cd*]indazol-9-0l, hydrochloride

A mixture of 3 g of 1-chloro-7-hydroxy-4-nitro-9H-xanthen-9-one, 2.7 g of 2-[(hydrazinoethyl)amino]ethanol, and 2 ml of diisopropylethylamine in 60 ml of *N,N*-dimethylformamide was stirred under an argon atmosphere for one hour. The solution was concentrated in vacuo and the residue was triturated with 2-propanol to leave a yellow crystalline solid. The solid was dissolved in minimal hot N,N-dimethylformamide and then acidified with a solution.of hydrogen chloride in 2-propanol to give 3.28 g of produce as a salt with 0.98 equivalents of hydrogen chloride, mp >300°C.

Example 16

5-Amino-2-[2-[(2-hydroxyethyl)amino]ethyl]-2*H*-[1]benzopyrano[4,3,2-*cd*]indazol-9-ol, hydrochloride

A mixture of 3.7 g of 2-[2-[(2-hydroxyethyl)amino]ethyl]-5-nitro-2*H*-[1]benzopyrano[4,3,2-*cd*]indazol-9-ol, hydrochloride and 0.25 g of 20% palladium on carbon in 300 ml of anhydrous methanol was stirred under hydrogen at atmospheric pressure until the hydrogen uptake measured 650 ml. The mixture was filtered and the filtrate concentrated in vacuo to dryness. The residue was dissolved in 100 ml of boiling methanol and the solution acidified with hydrogen chloride in 2-propanol. The solid was collected, washed with 2-propanol, and dried in vacuo at 60°C to give 2.1 g of product as a salt with 1.8 equivalents of hydrogen chloride and 0.88 equivalent of water mp 315°C with decomposition.

Example 17

2-[2-[(2-Hydroxyethyl)amino]ethyl]-9-hydroxy-2*H*-[1]benzopyrano[4,3,2-*cd*]indazol-5-yl]-2-[(2-hydroxyethyl)amino]acetamide, hydrochloride

A mixture of 10 g of 1-chloro-7-hydroxy-4-nitro-9H-xanthen-9-one, 20.1 g of *N*-(2-hydrazinoethyl)-*N*-[2-(phenylmethoxy)ethyl]benzenemethanamine and 6.0 ml of diisopropylethylamine in 200 ml of N,N-dimethylformamide was stirred under an argon atmosphere for two hours. The reaction was concentrated in vacuo to an oil which crystallized on standing. Trituration of the solid with 2-propanol, filtration, and drying at 60°C afforded 15.4 g of product. Recrystallization from methanol gave 5 - nitro - 2 - [2 - [[2 - (phenylmethoxy)ethyl] (phenylmethyl)amino]ethyl] - 2H - [1]benzopyrano[4,3,2 - *cd*]indazol - 9 - ol, mp 155—156°C after recrystallization from acetone.

A mixture of 10 g of 5 - nitro - 2 - [2 - [[2 - (phenylmethoxy)ethyl](phenylmethyl)amino]ethyl] - 2*H* - [1]benzopyrano[4,3,2 - *cd*]indazol - 9 - ol and 5 g of Raney nickel in 150 ml N,N-dimethylformamide was stirred at atmospheric pressure under hydrogen overnight. The mixture was filtered and the filtrate was concentrated in vacuo to a thick oil. The 5-amino-2-[[2-(phenylmethoxy)ethyl](phenylmethyl)amino]-ethyl] - 2*H* - [1]benzopyrano[4,3,2 - *cd*]indazol-9-ol so obtained was used without further processing.

To a solution of 9.44 g of the above oil in 220 ml of dry tetrahydrofuran was added 14.2 g of N-[2-(phenylmethoxy)ethyl]-N-(phenylmethyl)glycine and 4.3 g of N'-(ethylcarbonimidoyl)-N,N-dimethyl-1,3-propanediamine, monohydrochloride. The mixture was stirred under argon at 25°C for three hours. The reaction mixture was poured into 200 ml of aqueous saturated sodium bicarbonate solution and then extracted into dichloromethane. The extracts were concentrated in vacuo and the residue was triturated with acetone. The resulting solid was recrystallized from acetone to afford *N* - [9 - hydroxy - 2 - [2 - [[2 - (phenylmethoxy)ethyl](phenylmethyl)amino]ethyl] - 2*H* - [1]benzopyrano[4,3,2 - *cd*]indazol - 5 - yl] - 2 - [[2 - (phenylmethoxy)ethyl](phenylmethyl)amino]acetamide, mp 106—110°C.

A mixture of 2.5 g of the above acetamide and 0.20 g of 20% palladium hydroxide on carbon in 50 ml of glacial acetic acid was stirred under a hydrogen atmosphere at atmospheric pressure for 24 hours. The mixture was filtered through a celite pad and the filtrate was evaporated to an oily residue. The residue was dissolved in minimal methanol and then acidified with a solution of hydrogen chloride in 2-propanol. The precipitate was collected and dried in vacuo to give 2 - [2 - [(2 - hydroxyethyl)amino]ethyl] - 9 - hydroxy - 2*H* - [1]benzopyrano[4,3,2 - *cd*]indazol - 5 - yl] - 2 - [(2 - hydroxyethyl)amino]acetamide, hydrochloride, having 2.44 equivalents of hydrogen chloride and 2.61 equivalents of water, mp 223°C with decomposition.

Example 18

2-[2[(2-Hydroxyethyl)amino]ethyl]-5-[[2-[(2-hydroxyethyl)amino]ethyl]amino-2*H*-[1]benzopyrano[4,3,2-*cd*]indazol-9-ol, hydrochloride

A solution of 3.0 g of *N* - [9 - hydroxy - 2 - [2 - [[2 - (phenylmethoxy)ethyl](phenylmethyl)amino]-ethyl) - 2*H*-[1]benzopyrano[4,3,2 - *cd*]indazol - 5 - yl] - 2 - [[2 - (phenylmethoxy)ethyl](phenylmethyl)-amino]acetamide in 25 ml of tetrahydrofuran was treated with 15 ml of a 1 M solution of lithium tetrahydroaluminate in tetrahydrofuran. The reaction was heated under reflux for five hours and then cooled to 5°C. Saturated aqueous ammonium chloride (20 ml) was carefully added and the mixture stirred at 25°C overnight. The mixture was filterd, extracted with ethyl acetate, and the extract passed through a short column of silica gel with dichloromethane, affording a clear oil on evaporation. The oil crystallized from cold ether providing 2 - [2 - [[2 - (phenylmethoxy)ethyl](phenylmethyl)amino]ethyl - 5 - [[2 - [[2-(phenylmethoxy)ethyl](phenylmethyl)amino]ethyl]amino] - 2*H* - [1]benzopyrano[4,3,2 - *cd*]indazol - 9 - ol, mp 94—96°C.

A mixture of 2.0 g of the above product and 0.3 g of 20% palladium hydroxide on carbon in 50 ml of glacial acetic acid was stirred under hydrogen at atmospheric pressure for 24 hours. The mixture was filtered and the filtrate concentrated in vacuo to an oil. The oil was dissolved in 2-propanol and acidified with a solution of hydrogen chloride in 2-propanol. The resulting precipitate was collected and dried in vacuo at 56°C to give the title salt, containing 2.94 equivalents of hydrogen chloride and 0.35 equivalents of water, 0.79 equivalents of 2-propanol, mp 335°C with decomposition.

Example 19

5-Nitro-2-[2-(diethylamino)ethyl]-8-methoxy-2*H*-[1]benzopyrano[4,3,2-*cd*]indazol, hydrochloride

A mixture of 5.0 g of 1-chloro-6-methoxy-4-nitro-9*H*-xanthen-9-one, 2.58 g of [2-(diethylamino)-ethyl]hydrazine, and 3.43 ml of diisopropylethylamine in 100 ml of N,N-dimethylformamide was stirred for two hours. The mixture was evaporated to dryness and the residue was triturated with 2-propanol. The solid was collected, washed with diethylether, and dried at 60°C in vacuo to afford the title compound, a salt with 1.0 equivalent of hydrogen chloride, mp 285—288°C.

Example 20

5-Nitro-2-[2-(diethylamino)ethyl]-2*H*-[1]benzopyrano[4,3,2-*cd*]indazol-8-ol, hydrochloride

A mixture of 6.0 g of 1-chloro-6-hydroxy-4-nitro-9*H*-xanthen-9-one and 5.4 g of [2-(diethylamino)ethyl]-hydrazine and 3.9 ml of diisopropylethylamine in 100 ml of N,N-dimethylformamide was stirred at 25°C for three hours. The mixture was evaporated and the residue crystallized from 2-propanol to afford the title compound, a salt with one equivalent of hydrogen chloride, mp 285°C (decomposition).

Example 21

5-Amino-2-[2-(diethylamino)ethyl]-2*H*-[1]benzopyrano[4,3,2-*cd*]indazol-8-ol

A solution of 5.0 g of 5 - nitro - 2 - [2 - (diethylamino)ethyl] - 2*H* - [1] - benzopyrano[4,3,2 - *cd*]indazol - 8 - ol in 200 ml of DMF with 0.2 g of 20% palladium hydroxide on carbon was stirred under a hydrogen atmosphere for 24 hours. The mixture was filtered and the filtrate was evaporated in vacuo to leave the product 5 - amino - 2 - [2 - (diethylamino)ethyl] - 2*H* - [1]benzopyrano[4,3,2 - *cd*]indazol - 8 - ol, as an oily solid.

Preparation of Starting Materials

Example A

1-chloro-7-methoxy-4-nitro-9*H*-xanthen-9-one

2-(5-chloro-2-nitrophenoxy)-5-methoxybenzoic acid, 50 g, was added to 400 g of polyphosphoric acid, and the mixture stirred at 110°C for 24 hours. The mixture was poured into 2 liters of water, and the resulting precipitate filtered to afford a light green solid. Recrystallization from D.M.F. gave the title compound as green needles, mp 249—251°C.

Example B

2-(5-chloro-2-nitrophenoxy)-5-methoxybenzoic acid

To a stirred suspension of 65 g of 2-(5-chloro-2-nitrophenoxy)-5-methoxybenzoic acid, methyl ester, in 600 ml of methanol, was added 97.5 ml of 6N KOH solution. This suspension was stirred for 18 hours and the solution which resulted was neutralized with 200 ml of 10% HCl solution. The resulting precipitate was filtered, and washed with 400 ml of $H_2O$. Recrystallization from acetonitrile gave the title compound as a white solid, mp 232—234°C.

Example C

2-(5-chloro-2-nitrophenoxy)-5-methoxybenzoic acid, methyl ester

To a solution of 61.8 g of 2-hydroxy-5-methoxybenzoic acid, methyl ester in 700 ml DMF was added 14.96 g of a 60% suspension of sodium hydride in mineral oil. This was followed by addition of 50.88 g of sodium iodide, 64.64 g of copper(I)iodide, and 2.16 g of finely powdered copper metal. The mixture was heated to 85°C, for 15 minutes, then 71.7 g of 2,4-dichloronitrobenzene was added, and the reaction was

stirred at 85°C for 16 hours. The mixture was filtered, and the filtrate concentrated to an oily residue. Sodium thiosulfate, 400 ml of a 20% solution was added, followed by extraction with ethyl acetate (3 × 400 ml). The combined ethyl acetate extracts were washed with water (2 × 325 ml), dried over anhydrous sodium sulfate, then evaporated to afford a pale yellow solid, which was triturated with cyclohexane and filtered giving the title compound, mp 114—116°C.

Example D

2-Hydroxy-5-methoxybenzoic acid, methyl ester

A mixture of 73 g of 5-methoxysalicylic acid, and 95 ml of concentrated hydrochloric acid in 700 ml of methanol was heated under reflux for 48 hours. Potassium carbonate (10 g) was added, and the mixture concentrated to an oily residue which was dissolved in 250 ml of ethyl acetate. This solution was washed with two 100 ml portions of 10% sodium bicarbonate solution, then dried over anhydrous sodium sulfate. Evaporation of solvent afforded the title compound as an amber oil.

Example E

1-chloro-7-hydroxy-4-nitro-9*H*-xanthen-9-one

A mixture of 10 g of 1-chloro-7-methoxy-4-nitro-9*H*-xanthen-9-one and 84 ml of a 1 M solution of boron tribromide in dichloromethane in 280 ml of dichloromethane was stirred at room temperature under an argon atmosphere for 16 hours, and then it was treated with 100 ml of methanol, and stirred for three hours. The mixture was filtered to give a light green solid. Recrystallization from chloroform-methanol afforded the title compound, mp 290—292°C.

Example F

2-(5-Chloro-2-nitrophenoxy)-4-methoxybenzoic acid, methyl ester

To a stirred solution of 15 g of methyl 2-hydroxy-4-methoxy-benzoate in 185 ml of dimethylformamide under an argon was added 3.63 g of a 60% dispersion of sodium hydride in oil. To this slurry was added 12.35 g of sodium iodide, 15.69 g of copper(I)iodide, and 0.525 g of copper powder. The mixture was heated for 15 minutes at 85°C and 17.4 g of 2,4-dichloronitrobenzene was added. After 48 hours at 85°C, the reaction was cooled to 25°C and methanol was added. The reaction was filtered and the filtrate was concentrated in vacuo to about 50 ml then diluted with 150 ml of ethylacetate. The solution was washed with a 20% aqueous solution of sodium thiosulfate and the organic portion was separated and dried over sodium sulfate. The solvent was evaporated to leave a tan solid. Recrystallization from ethyl acetate gave the title compound, mp 121—123°C.

Example G

1-Chloro-6-methoxy-4-nitro-9*H*-xanthen-9-one

To 500 g of polyphosphoric acid stirred at 110°C was added 62.9 g of 2-(5-chloro-2-nitrophenoxy)-4-methoxybenzoic acid, methyl ester. After 18 hours, the hot solution was poured into water, and the resulting precipitate collected. The solid was washed with water and isopropyl alcohol to give 57.9 g of the title compound, mp 211—213°C after recrystallization from DMF.

Example H

1-Chloro-6-hydroxy-4-nitro-9*H*-xanthen-9-one

A mixture of 20.0 g of 1-chloro-6-methoxy-4-nitro-9*H*-xanthen-9-one and 27.04 g of anhydrous powdered aluminium chloride was heated under reflux in 400 ml of 1,2-dichloroethane under an argon atmosphere for three hours. The liquid portion of the reaction mixture was decanted and evaporated to a dark residue. Both residues were combined and treated with 200 ml of concentrated hydrochloric acid for one hour. The solid was collected and washed with water and 2-propanol. The grey solid was dried in vacuo at room temperature to afford the title compound, mp 179—182°C.

Example I

1-(2-Hydrazinoethyl)pyrrolidine

A mixture of 200 g of 85% hydrazine hydrate, 200 ml of water, 170 g of *N*-chloroethylpyrrolidine hydrochloride, and 70 g of potassium carbonate was boiled under reflux for seven hours. Sodium hydroxide (390 g) was added and the mixture was extracted with ether. The ethereal extract, dried and distilled, yielded the title compound, bp 107—111°C 25 mbar (18.5 mm).

Example J

N-[2-(Phenylmethoxy)ethyl]-N-(phenylmethyl)glycine, hydrobromide

A mixture of 13.8 g of bromoacetic acid, 200 ml of acetonitrile, 19.0 ml of *N,N*-diisopropylethylamine, and 26.3 g of *N,O*-dibenzylethanolamine was heated 24 hours under reflux. An additional portion of bromoacetic acid (3.5 g) was added, heating continued 24 hours. Refrigeration of the resulting mixture caused precipitation of the title compound, white needles of mp 115—117°C after recrystallization from ethyl acetate.

Example K

N-(2-Hydrazinoethyl)-N-[2-(phenylmethoxy)ethyl]benzenemethanamine

A solution of 10.0 g (0.033 mole) of *N*-(2-chloroethyl)-*N*-[2-(phenylmethoxy)ethyl]benzene-methanamine [Nodor, et al, *Acta Chim. Acad. Sci. Hung.,* 2:152 (1952)], 57.2 g (1.65 mol) of anhydrous hydrazine, and 210 ml of absolute ethanol was stirred at 25°C for 18 hours. The mixture was poured into cold water and the solution thrice extracted with 500-ml portions of ether. The combined ether extracts were dried over anhydrous magnesium sulfate and concentrated at a temperature below 35°C to give 7.2 g of the product as an unstable oil.

**Claims for the Contracting States: BE CH DE FR GB IT LI LU NL SE**

1. A benzopyrano[4,3,2-*cd*]indazole compound having in free base form the following general formula I

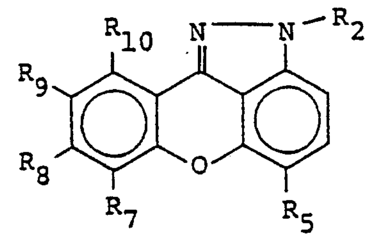

I

and the pharmaceutically acceptable salt thereof, wherein:

$R_7$, $R_8$, $R_9$ and $R_{10}$ independently represent hydrogen, hydroxy or alkoxy of from one to four carbon atoms;

$R_2$ is ANR'R'' in which A is a straight or branched alkylene chain of from 2 to 5 carbon atoms, optionally substituted with hydroxyl; and in which R' and R'' are independently hydrogen, a straight or branched alkyl of from one to four carbon atoms, optionally substituted with hydroxyl, or R' and R'', when taken together, represent the bifunctional radical

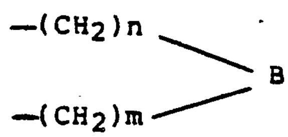

in which n and m are, independently, two or three and B is a direct bond or O, S, or NR''' wherein R''' is hydrogen or straight or branched alkyl of from one to four carbon atoms, optionally substituted with hydroxyl; and

$R_5$ is nitro, $NH_2$, $NHR_2$ wherein $R_2$ is defined above or NHR'''' wherein R'''' is a radical having the formula

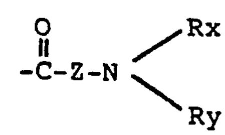

in which Z is a straight or branched alkylene of from one to four carbon atoms and $R_x$ and $R_y$ are each independently hydrogen or straight or branched alkyl of from one to four carbon atoms optionally substituted with hydroxyl, or R'''' is the radical

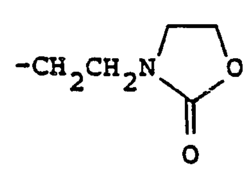

2. A benzopyrano[4,3,2-*cd*]indazole compound according to Claim 1, having in free base form the following general formula:

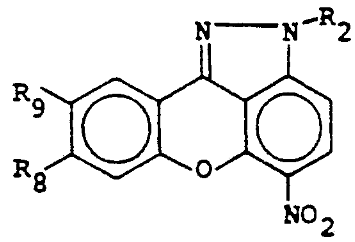

and the pharmaceutically acceptable salts thereof; wherein $R_2$, $R_8$ and $R_9$ are as defined in Claim 1.

3. A benzopyrano[4,3,2-*cd*]indazole compound according to Claim 1, having in free base form the following general formula:

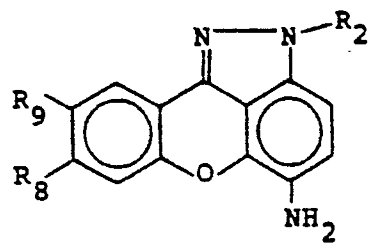

and the pharmaceutically acceptable salts thereof; wherein $R_2$, $R_8$ and $R_9$ are as defined in Claim 1.

4. A benzopyrano[4,3,2-*cd*]indazole compound according to Claim 1, having in free base form the following general formula:

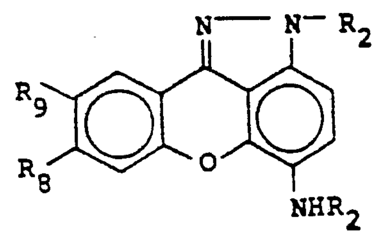

and the pharmaceutically acceptable salts thereof; wherein $R_2$, $R_8$ and $R_9$ are as defined in Claim 1.

5. A compound according to Claim 1 or 4, wherein $R_2$ is diethyl aminoethyl and $R_5$ is $NHCH_2CH_2NH_2$.

6. A compound according to Claim 1 chosen from:

N-[2-[2-(diethylamino)-ethyl]-2*H*-[1]-benzopyrano[4,3,2-*cd*]indazol-5-yl]ethanediamine;

2-[3-(dimethylamino)propyl]-5-nitro-2*H*-[1]benzopyrano[4,3,2-*cd*]indazol-9-ol; and

2-[2-(diethylamino)ethyl]-5-[[2-[(2-hydroxyethyl)amino]ethyl]amino-2*H*-[1]benzopyrano[4,3,2-*cd*]indazol-9-ol.

7. A compound having the following general formula:

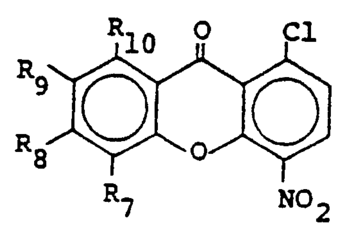

wherein $R_7$, $R_8$, $R_9$ and $R_{10}$ are hydrogen, hydroxy or $C_{1-4}$ alkoxy.

8. A compound according to Claim 7, having the following general formula:

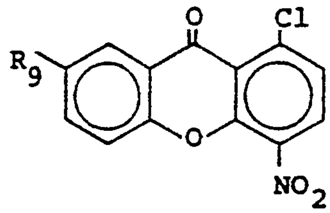

wherein $R_9$ is as defined in Claim 7.

9. A process for preparing a benzopyrano[4,3,2-*cd*]indazole compound having the following general formula:

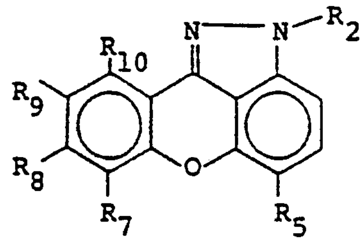

which comprises:

reacting an appropriately substituted 1-chloro-4-nitro-9*H*-xanthen-9-one and an $R_2$-substituted hydrazine to form the compound as claimed in Claim 1 wherein $R_5$ is nitro;

if desired, converting said compound, wherein $R_5$ is nitro by reduction, to a compound as claimed in Claim 1 wherein $R_5$ is $NH_2$;

if further desired, converting the resulting compound wherein $R_5$ is $NH_2$ to a compound as claimed in Claim 1, wherein $R_5$ is $NHR_2$ either (i) by alkylation with a haloalkylamine having the formula $XR_2$ (optionally after covering sensitive groups with protecting groups), (ii) by reaction with an aldehyde or acetal, ketone or ketal and reducing the resulting Schiff base, or (iii) by monoacylating with a reactive derivative of an acylating agent corresponding to R'''' and reducing the acylated product (optionally after covering sensitive groups with protecting groups);

if desired, removing the protecting groups by hydrolysis or reduction; and

isolating the product in free base or acid addition salt form;

wherein X is chloro or bromo and $R_2$, $R_5$, $R_7$, $R_8$, $R_9$, $R_{10}$ and R'''' are as defined in Claim 1.

10. A pharmaceutical composition comprising a compound as claimed in any one of Claims 1 to 6 in combination with a pharmaceutically acceptable carrier or diluent.

**Claims for the Contracting State: AT**

1. A process for preparing a benzopyrano[4,3,2-*cd*]indazole compound having in free base form the following general formula I

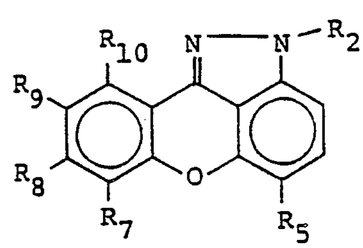

I

and the pharmaceutically acceptable salts thereof, wherein:

$R_7$, $R_8$, $R_9$ and $R_{10}$ independently represent hydrogen, hydroxy or alkoxy of from one to four carbon atoms;

$R_2$ is ANR'R'' in which A is a straight or branched alkylene chain of from 2 to 5 carbon atoms, optionally substituted with hydroxyl; and in which R' and R'' are independently hydrogen, a straight or branched alkyl of from one to four carbon atoms, optionally substituted with hydroxyl, or R' and R'', when taken together, represent the bifunctional radical

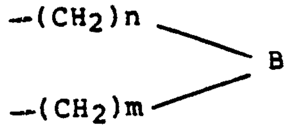

in which n and m are, independently, two or three and B is a direct bond or O, S, or NR''' wherein R''' is hydrogen or straight or branched alkyl of from one to four carbon atoms, optionally substituted with hydroxyl; and

$R_5$ is nitro, $NH_2$, $NHR_2$ wherein $R_2$ is defined above or NH'R'''' wherein R'''' is a radical having the formula

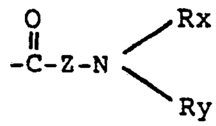

in which Z is a straight or branched alkylene of from one to four carbon atoms and $R_x$ and $R_y$ are each independently hydrogen or straight branched alkyl of from one to four carbon atoms optionally substituted with hydroxyl, or R'''' is the radical

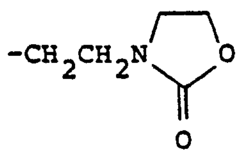

which process comprises:

reacting an appropriately substituted 1-chloro-4-nitro-9*H*-xanthen-9-one and an $R_2$-substituted hydrazine to form the compound of formula I, wherein $R_5$ is nitro;

if desired, converting said compound, wherein $R_5$ is nitro by reduction, to a compound of formula I wherein $R_5$ is $NH_2$;

if further desired, converting the resulting compound wherein $R_5$ is $NH_2$ to a compound of formula I, wherein $R_5$ is $NHR_2$ either (i) by alkylation with a haloalkylamine having the formula $XR_2$ (optionally after covering sensitive groups with protecting groups), (ii) by reaction with an aldehyde or acetal, ketone or ketal and reducing the resulting Schiff base, or (ii) by monoacylating with a reactive derivative of an acylating agent corresponding to R'''' and reducing the acylated product (optionally after covering sensitive groups with protecting groups);

if desired, removing the protecting groups by hydrolysis or reduction; and

isolating the product in free base or acid addition salt form;

wherein X is chloro or bromo and $R_2$, $R_5$, $R_7$, $R_8$, $R_9$, $R_{10}$ and R'''' are as defined above.

2. A process according to Claim 1 for preparing a benzopyrano[4,3,2-*cd*]indazole having in free base form the following general formula:

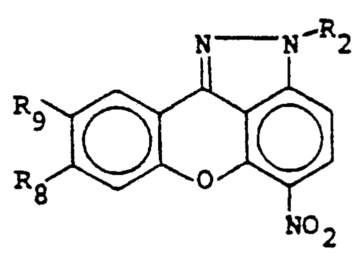

and the pharmaceutically acceptable salts thereof; wherein $R_2$, $R_8$ and $R_9$ are as defined in Claim 1.

3. A process according to Claim 1 for preparing a benzopyrano[4,3,2-*cd*]indazole having in free base form the following general formula:

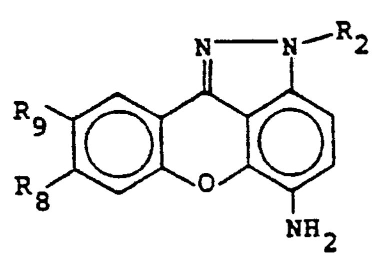

and the pharmaceutically acceptable salts thereof; wherein $R_2$, $R_8$ and $R_9$ are as defined in Claim 1.

4. A process according to Claim 1 for preparing a benzopyrano[4,3,2-*cd*]indazole having in free base form the following general formula:

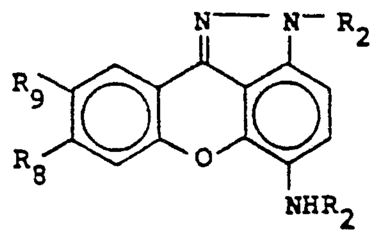

and the pharmaceutically acceptable salts thereof; wherein $R_2$, $R_8$ and $R_9$ are as defined in Claim 1.

5. A compound according to Claim 1 or 4, wherein $R_2$ is diethyl aminoethyl and $R_5$ is $NHCH_2CH_2NH_2$.

6. A process according to Claim 1, in which a compound having one of the following names is prepared:

N-[2-[2-(diethylamino)-ethyl]-2*H*-[1]-benzopyrano[4,3,2-*cd*]indazol-5-yl]ethanediamine;

2-[3-(dimethylamino)propyl]-5-nitro-2*H*-[1]benzopyrano[4,3,2-*cd*]indazol-9-ol; and

2-[2-(diethylamino)ethyl]-5-[[2-[(2-hydroxyethyl)amino]ethyl]amino-2*H*-[1]benzopyrano[4,3,2-*cd*]indazol-9-ol

7. A process for preparing a compound having the following general formula:

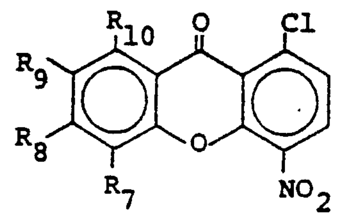

which comprises non-oxidatively dehydrating a compound having the formula:

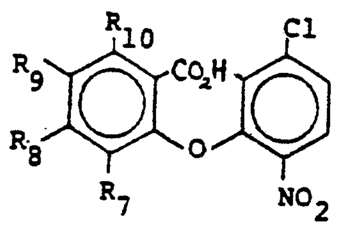

wherein $R_7$, $R_8$, $R_9$ and $R_{10}$ are as defined in Claim 1.

8. A process according to Claim 7, wherein $R_7$, $R_8$ and $R_{10}$ are hydrogen.

9. A process for preparing a pharmaceutical composition, which process comprises combining a compound prepared by a process as claimed in any one of Claims 1 to 6 with a pharmaceutically acceptable carrier or diluent.

**Patentansprüche für die Vertragsstaaten: BE CH DE FR GB IT LI LU NL SE**

1. Benzopyrano-[4,3,2-cd]-indazolverbindung, die in Form der freien Base die folgende allgemeine Formel I hat

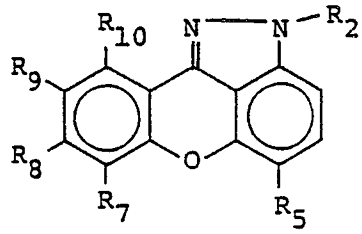

I

und die pharmazeutisch akzeptablen Salze derselben, worin:

$R_7$, $R_8$, $R_9$ und $R_{10}$ unabhängig voneinander Wasserstoff, Hydroxy oder Alkoxy mit ein bis vier Kohlenstoffatomen darstellen;

$R_2$ für ANR'R'' steht, worin A eine gerade oder verzweigte Alkylenkette mit zwei bis fünf Kohlenstoffatomen, gegebenenfalls mit Hydroxyl substituiert, ist; und worin R' und R'' unabhängig voneinander Wasserstoff, ein gerades oder verzweigtes Alkyl mit einem bis vier Kohlenstoffatomen, gegebenenfalls mit Hydroxyl substituiert, bedeuten, oder R' und R'', zusammengenommen, die bifunktionelle Gruppe

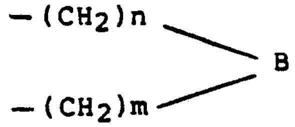

darstellen, worin n und m unabhängig voneinander 2 oder 3 bedeuten und B eine direkte Bindung oder O, S oder NR''' ist, worin R''' für Wasserstoff oder ein gerades oder verzweigtes Alkyl mit einem bis vier Kohlenstoffatomen, gegebenenfalls mit Hydroxyl substituiert, steht; und

$R_5$ Nitro, $NH_2$, $NHR_2$, worin $R_2$ oben definiert ist, oder NHR'''' darstellt, worin R'''' eine Gruppe der Formel

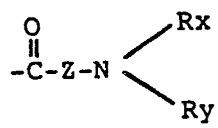

bedeutet, in welcher z ein gerades oder verzweigtes Alkylen mit einem bis vier Kohlenstoffatomen ist, und $R_x$ und $R_y$ jeweils unabhängig Wasserstoff oder gerades oder verzweigtes Alkyl mit einem bis vier Kohlenstoffatomen, gegebenenfalls mit Hydroxyl substituiert, darstellen, oder R'''' die Gruppe

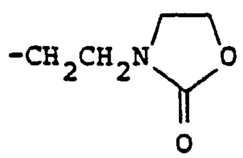

ist.

2. Benzopyrano-[4,3,2-cd]-indazolverbindung nach Anspruch 1, die in Form der freien Base die folgende allgemeine Formel hat:

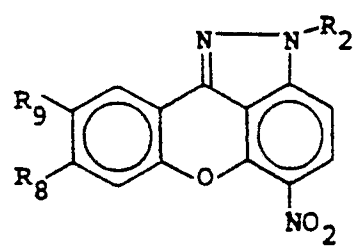

und die pharmazeutisch akzeptablen Salze derselben; worin $R_2$, $R_8$ und $R_9$ wie in Anspruch 1 definiert sind.

3. Benzopyrano-[4,3,2-cd]-indazolverbindung nach Anspruch 1, die in Form der freien Base die folgende allgemeine Formel hat:

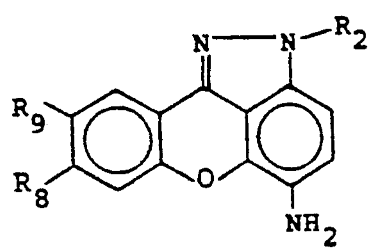

und die pharmazeutisch akzeptablen Salze derselben; worin $R_2$, $R_8$ und $R_9$ wie in Anspruch 1 definiert sind.

4. Benzopyrano-[4,3,2-cd]-indazolverbindung nach Anspruch 1, die in Form der freien Base die folgende allgemeine Formel hat:

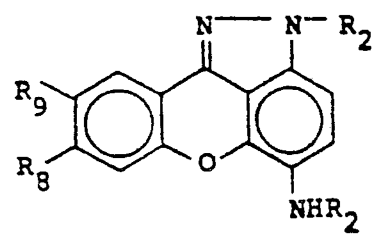

und die pharmazeutisch akzeptablen Salze derselben; worin $R_2$, $R_8$ und $R_9$ wie in Anspruch 1 definiert sind.

5. Verbindung nach Anspruch 1 oder 4, worin $R_2$ Diethylaminoethyl ist und $R_5$ für $NHCH_2CH_2NH_2$ steht.

6. Verbindung nach Anspruch 1, ausgewählt aus:

N-[2-[2-(Diethylamino)-ethyl]-2H-[1]-benzopyrano-[4,3,2-cd]-indazol-5-yl]-ethandiamin;

2-[3-(Dimethylamino)-propyl]-5-nitro-2H-[1]-benzopyrano-[4,3,2-cd]-indazol-9-ol; und

2-[2-(Diethylamino)-ethyl]-5-[[2-[(2-hydroxyethyl)-amino]ethyl]-amino-2H-[1]-benzopyrano-[4,3,2-cd]-indazol-9-ol.

7. Verbindung der allgemeinen Formel:

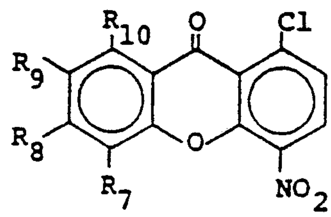

worin $R_7$, $R_8$, $R_9$ und $R_{10}$ Wasserstoff, Hydroxy oder $C_{1-4}$-Alkoxy bedeuten.

8. Verbindung nach Anspruch 7 der allgemeinen Formel:

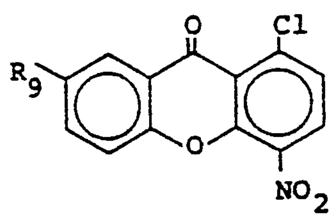

worin $R_9$ wie in Anspruch 7 definiert ist.

9. Verfahren zur Herstellung einer Benzopyrano-[4,3,2-cd]-indazolverbindung der allgemeinen Formel:

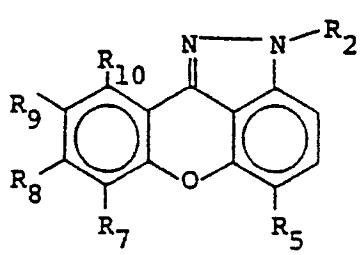

welches umfaßt:

Umsetzung eines entsprechend substituierten 1-Chlor-4-nitro-9H-xanthen-9-ons und eines $R_2$-substituierten Hydrazins zur Bildung der in Anspruch 1 beanspruchten Verbindung, worin $R_5$ Nitro ist;

gewünschtenfalls Umwandlung dieser Verbindung, worin $R_5$ Nitro ist, durch Reduktion zu einer Verbindung, wie in Anspruch 1 beansprucht, worin $R_5$ für $NH_2$ steht;

weiters gewünschtenfalls Umwandlung der resultierenden Verbindung, worin $R_5$ für $NH_2$ steht, in eine Verbindung, wie in Anspruch 1 beansprucht, worin $R_5$ für $NHR_2$ steht, entweder (i) durch Alkylierung mit einem Halogenalkylamin der Formel $XR_2$ (gegebenenfalls nach Bedecken empfindlicher Gruppen mit Schutzgruppen), (ii) durch Reaktion mit einem Aldehyd oder Acetal, Keton oder Ketal und Reduktion der

resultierenden Schiffschen Base, oder (iii) durch Monoacylierung mit einem reaktiven Derivat eines Acylierungsmittels entsprechend R'''' und Reduktion des acylierten Produkts (gegebenenfalls nach Bedecken empfindlicher Gruppen mit Schutzgruppen);

gewünschtenfalls Entfernung der Schutzgruppen mittels Hydrolyse oder Reduktion; und

Isolierung des Produkts in Form der freien Base oder des Säureadditionssalzes;

worin X Chlor oder Brom ist und $R_2$, $R_5$, $R_7$, $R_8$, $R_9$, $R_{10}$ und R'''' wie in Anspruch 1 definiert sind.

10. Pharmazeutische Zusammensetzung, die eine Verbindung nach einem der Ansprüche 1 bis 6 in Kombination mit einem pharmazeutisch akzeptablen Träger oder Verdünnungsmittel umfaßt.

**Patentansprüche für den Vertragsstaat: AT**

1. Verfahren zur Herstellung einer Benzopyrano-[4,3,2-cd]-indazolverbindung, die in Form der freien Base die folgende allgemeine Formel I hat

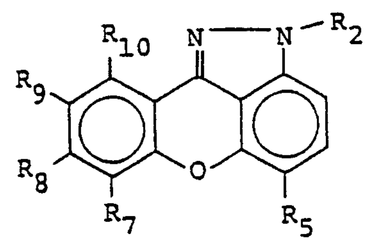

I

und die pharmazeutisch akzeptablen Salze derselben, worin:

$R_7$, $R_8$, $R_9$ und $R_{10}$ unabhängig voneinander Wasserstoff, Hydroxy oder Alkoxy mit ein bis vier Kohlenstoffatomen darstellen;

$R_2$ für ANR'R'' steht, worin A eine gerade oder verzweigte Alkylenkette mit zwei bis fünf Kohlenstoffatomen, gegebenenfalls mit Hydroxyl substituiert, ist; und worin R' und R'' unabhängig voneinander Wasserstoff, ein gerades oder verzweigtes Alkyl mit einem bis vier Kohlenstoffatomen, gegebenenfalls mit Hydroxyl substituiert, bedeuten, oder R' und R'', zusammengenommen, die bifunktionelle Gruppe

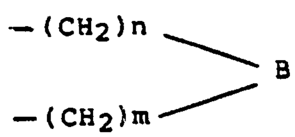

darstellen, worin n und m unabhängig voneinander 2 oder 3 bedeuten und B eine direkte Bindung oder O, S oder NR''' ist, worin R''' für Wasserstoff oder ein gerades oder verzweigtes Alkyl mit einem bis vier Kohlenstoffatomen, gegebenenfalls mit Hydroxyl substituiert, steht; und

$R_5$ Nitro, $NH_2$, $NHR_2$, worin $R_2$ oben definiert ist, oder NHR'''' darstellt, worin R'''' eine Gruppe der Formel

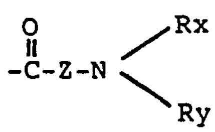

bedeutet, in welcher z ein gerades oder verzweigtes Alkylen mit einem bis vier Kohlenstoffatomen ist, und $R_x$ und $R_y$ jeweils unabhängig Wasserstoff oder gerades oder verzweigtes Alkyl mit einem bis vier Kohlenstoffatomen, gegebenenfalls mit Hydroxyl substituiert, darstellen, oder R'''' die Gruppe

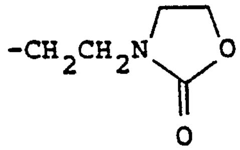

ist.

welches Verfahren umfaßt:

Umsetzung eines entsprechend substituierten 1-Chlor-4-nitro-9H-xanthen-9-ons und eines $R_2$-substituierten Hydrazins zur Bildung der Verbindung der Formel I, worin $R_5$ Nitro ist;

gewünschtenfalls Umwandlung dieser Verbindung, worin $R_5$ Nitro ist, durch Reduktion zu einer Verbindung der Formel I, worin $R_5$ für $NH_2$ steht;

weiters gewünschtenfalls Umwandlung der resultierenden Verbindung, worin $R_5$ für $NH_2$ steht, in eine Verbindung der Formel I, worin $R_5$ für $NHR_2$ steht, entweder (i) durch Alkylierung mit einem Halogenalkylamin der Formel $XR_2$ (gegebenenfalls nach Bedecken empfindlicher Gruppen mit Schutzgruppen), (ii) durch Reaktion mit einem Aldehyd oder Acetal, Keton oder Ketal und Reduktion der resultierenden Schiffschen Base, oder (iii) durch Monoacylierung mit einem reaktiven Derivat eines Acylierungsmittels entsprechend R'''' und Reduktion des acylierten Produkts (gegebenenfalls nach Bedecken empfindlicher Gruppen mit Schutzgruppen);

gewünschtenfalls Entfernung der Schutzgruppen mittels Hydrolyse oder Reduktion; und

Isolierung des Produkts in Form der freien Base oder des Säureadditionssalzes;

worin X Chlor oder Brom ist und $R_2$, $R_5$, $R_7$, $R_8$, $R_9$, $R_{10}$ und R'''' wie oben definiert sind.

2. Verfahren nach Anspruch 1 zur Herstellung eines Benzopyrano-[4,3,2-cd]-indazols, das in Form der freien Base die folgende allgemeine Formel hat:

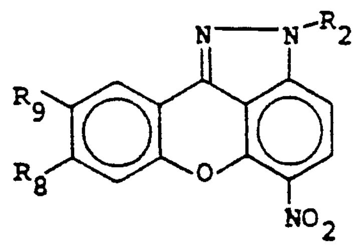

und die pharmazeutisch akzeptablen Salze davon; worin $R_2$, $R_8$ und $R_9$ wie in Anspruch 1 definiert sind.

3. Verfahren nach Anspruch 1 zur Herstellung eines Benzopyrano-[4,3,2-cd]-indazols, das in Form der freien Base die folgende allgemeine Formel hat:

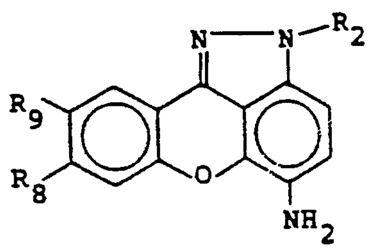

und der pharmazeutisch akzeptablen Salze davon; worin $R_2$, $R_8$ und $R_9$ wie in Anspruch 1 definiert sind.

4. Verfahren nach Anspruch 1 zur Herstellung eines Benzopyrano-[4,3,2-cd]-indazols, das in Form der freien Base die folgende allgemeine Formel hat:

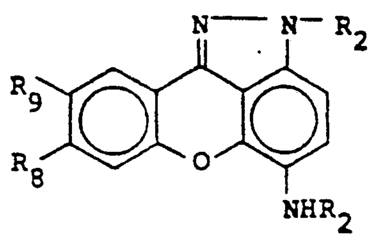

und der pharmazeutisch akzeptablen Salze davon; worin $R_2$, $R_8$ und $R_9$ wie in Anspruch 1 definiert sind.

5. Verfahren nach Anspruch 1 oder 4, worin $R_2$ Diethylaminoethyl ist und $R_5$ für $NHCH_2CH_2NH_2$ steht.

6. Verfahren nach Anspruch 1, bei welchem eine Verbindung mit einer der folgenden Bezeichnungen hergestellt wird:

N-[2-[2-(Diethylamino)-ethyl]-2H-[1]-benzopyrano-[4,3,2-cd]-indazol-5-yl]-ethandiamin;

2-[3-(Dimethylamino)-propyl]-5-nitro-2H-[1]-benzopyrano-[4,3,2-cd]-indazol-9-ol; und

2-[2-(Diethylamino)-ethyl]-5-[[2-[(2-hydroxyethyl)-amino]ethyl]-amino-2H-[1]-benzopyrano-[4,3,2-cd]-indazol-9-ol.

7. Verfahren zur Herstellung einer Verbindung der folgenden allgemeinen Formel:

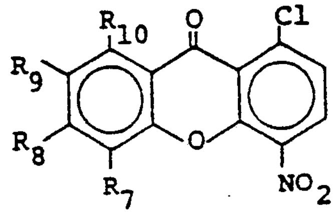

welches die nicht-oxidative Dehydratisierung einer Verbindung der Formel:

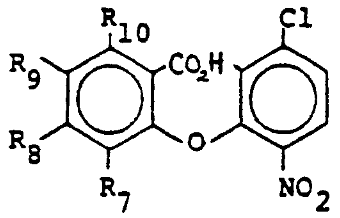

worin $R_7$, $R_8$, $R_9$ und $R_{10}$ wie in Anspruch 1 definiert sind, umfaßt.

8. Verfahren nach Anspruch 7, worin $R_7$, $R_8$ und $R_{10}$ Wasserstoff sind.

9. Verfahren zur Herstellung einer pharmazeutischen Zusammensetzung, welches Verfahren die Vereinigung einer mittels eines Verfahren nach einem der Ansprüche 1 bis 6 hergestellten Verbindung mit einem pharmazeutisch akzeptablen Träger oder Verdünnungsmittel umfaßt.

**Revendications pour les Etats contractants: BE CH DE FR GB IT LI LU NL SE**

1. Un composé de benzopyrano[4,3,2-cd]indazole ayant sous forme de base libre la formule générale I suivante:

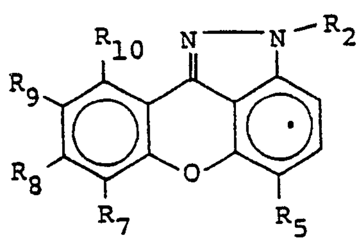

I

ainsi qui les sels pharmaceutiquement acceptables en dérivant, dans laquelle:

$R_7$, $R_8$, $R_9$ et $R_{10}$, indépendamment, représentent un atome d'hydrogène ou un groupe hydroxy ou alkoxy comprenant de 1 à 4 atomes de carbone;

$R_2$ est ANR'R'' avec A étant une chaîne alkyle linéaire ou ramifiée comprenant de 2 à 5 atomes de carbone, éventuellement substitué par un groupe hydroxyle et dans laquelle R' et R'' sont, indépendamment l'un de l'autre, un atome d'hydrogène ou un radical alkyle linéaire ou ramifié comprenant de 1 à 4 atomes de carbone, éventuellement substitué par un groupe hydroxyle, R' et R'', pris ensemble, présente le radical bifonctionnel:

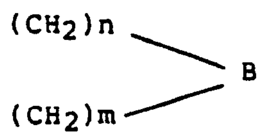

dans lequel:

n et m sont, indépendamment l'un de l'autre, égaux à 2 ou 3; et

B est une liaison directe ou est un atome d'oxygène, de soufre ou un groupe NR''' dans lequel R''' est un atome d'hydrogène ou un radical alkyle linéaire ou ramifié comprenant de 1 à 4 atomes de carbone, éventuellement substitué par un groupe hydroxyle; et

$R_5$ est un groupe nitro, $NH_2$, $NHR_2$ dans lequel $R_2$ est tel que défini ci-dessus ou NHR'''' dans lequel R'''' est un radical présentant la formule suivante:

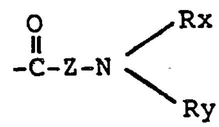

dans laquelle:

Z est un radical alkylène linéaire ou ramifié comprenant de 1 à 4 atomes de carbone; et

$R_x$ et $R_y$ sont, indépendamment l'un de l'autre, un atome d'hydrogène ou un radical alkyle linéaire ou ramifié comprenant de 1 à 4 atomes de carbone, éventuellement substitué par un groupe hydroxy, ou R'''' est le radical:

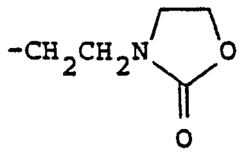

2. Un composé de benzopyrano[4,3,2-cd]indazole selon la revendication 1, ayant sous forme de base libre la formule générale suivante:

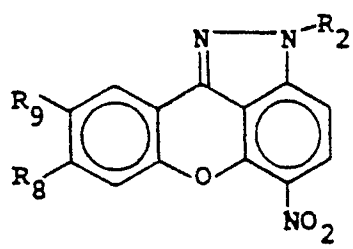

ainsi que les sels pharmaceutiquement acceptables en dérivant, dans laquelle $R_2$, $R_8$ et $R_9$ sont tels que définis dans la revendication 1.

3. Un composé de benzopyrano[4,3,2-cd]indazole selon la revendication 1, ayant sous forme de base libre la formule générale suivante:

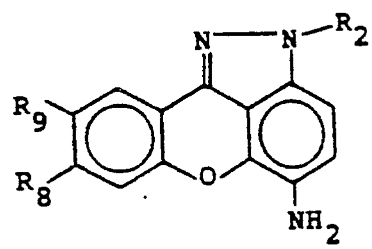

ainsi que les sels pharmaceutiquement acceptables en dérivant, dans laquelle $R_2$, $R_8$ et $R_9$ sont tels que définis dans la revendication 1.

4. Un composé de benzopyrano[4,3,2-cd]indazole selon la revendication 1, ayant sous forme de base libre la formule générale suivante:

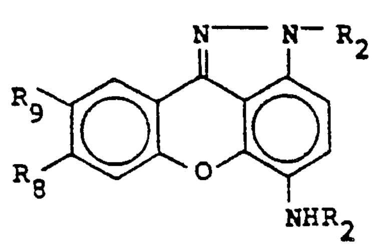

ainsi que le sels pharmaceutiquement acceptables en dérivant, dans laquelle $R_2$, $R_8$ et $R_9$ sont tels que définis dans la revendication 1.

5. Un composé selon la revendication 1 ou la revendication 4, dans lequel $R_2$ est un radical diéthylaminoéthyl et $R_5$ est $NHCH_2CH_2NH_2$.

6. Un composé selon la revendication 1, choisi parmi ceux appartenant à la liste suivante:
N-[2-[2-(diéthylamino)éthyl]-2H-[1]-benzopyrano[4,3,2-cd]indazol-5-yl]éthanediamine;
2-[3-(diméthylamino)propyl]-5-nitro-2H-[1]-benzopyrano-[4,3,2-cd]indazol-9-ol; et
2-[2-(diéthylamino)éthyl]-5-[[2-[(2-hydroxyéthyl)amino]éthyl]amino-2H-[1]benzopyrano[4,3,2-cd]indazol-9-ol.

7. Un composé présent la formule générale suivante:

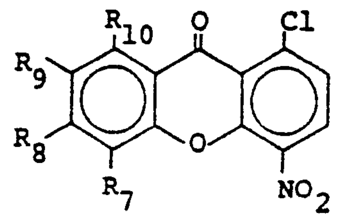

dans laquelle:

$R_7$, $R_8$, $R_9$ et $R_{10}$ sont un atome d'hydrogène ou un radical hydroxy ou alkoxy comprenant de 1 à 4 atomes de carbone.

8. Un composé selon la revendication 7, présentant la formule générale suivante:

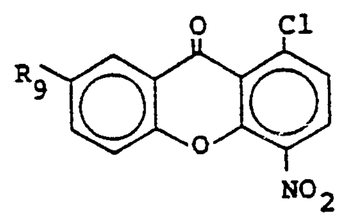

dans laquelle $R_9$ est tel que défini dans la revendication 7.

9. Un procédé de préparation d'un dérivé de benzopyrano[4,3,2-cd]indazole présentant la formule générale suivante:

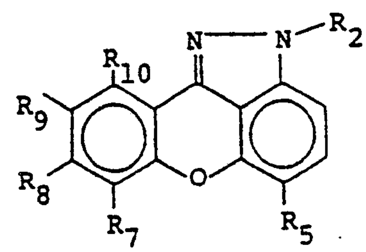

qui consiste:

à faire réagir un 1-chloro-4-nitro-9H-xanthèn-9-one convenablement substitué et un hydrazine substituée en $R_2$ pour former le composé ainsi que revendiqué dans la revendication 1, dans lequel $R_5$ est le groupe nitro;

le cas échéant, à convertir ledit composé dans lequel $R_5$ est le groupe nitro par réduction en un composé ainsi que revendiqué dans le revendication 1, dans lequel $R_5$ est $NH_2$;

le cas échéant encore, à convertir le composé résultant dans lequel $R_5$ est $NH_2$ en un composé ainsi que revendiqué dans la revendication 1, dans lequel $R_5$ est $NHR_2$ soit (i) par alkylation avec une halo-alkylamine présentant la formule $XR_2$ (éventuellement après protection des groupes sensibles à l'aide de groupes protecteurs), (ii) par réaction avec un aldéhyde, un acétal, une cétone ou un cétal et réduction de la base de Schiff résultante, ou (iii) par monoacylation à l'aide d'un dérivé réactif d'un agent d'acylation correspondant à R'''' et à réduire le produit acylé (éventuellement après protection des groupes sensibles à l'aide de groupes protecteurs);

le cas échéant, à éliminer les groupes protecteurs par hydrolyse ou réduction et à isoler le produit sous forme de base libre ou sous forme d'un sel d'addition d'acide, dans lequel X est un atome de chlore ou de brome et $R_2$, $R_5$, $R_7$, $R_8$, $R_9$, $R_{10}$ et R'''' sont tels que définis dans la revendication 1.

10. Une composition pharmaceutique comprenant un composé ainsi que revendiqué dans l'une quelconque des revendications 1 à 6, en association avec un support ou diluant pharmaceutiquement acceptable.

**Revendications pour l'Etat contractant: AT**

1. Un procédé de préparation d'un composé de benzopyrano[4,3,2-cd]indazole ayant sous forme de base libre la formule générale I suivante:

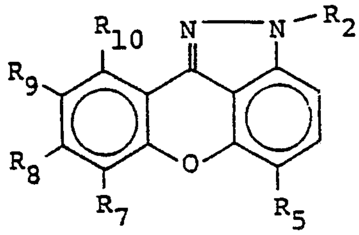

I

ainsi que les sels pharmaceutiquement acceptables en dérivant, dans laquelle:

$R_7$, $R_8$, $R_9$ et $R_{10}$, indépendamment, représentent un atome d'hydrogène ou un groupe hydroxy ou alkoxy comprenant de 1 à 4 atomes de carbone;

$R_2$ est ANR'R'' avec A étant une chaîne alkyle linéaire ou ramifiée comprenant de 2 à 5 atomes de carbone, éventuellement substitué par un groupe hydroxyle et dans laquelle R' et R'' sont, indépendamment l'un de l'autre, un atome d'hydrogène ou un radical alkyle linéaire ou ramifié comprenant de 1 à 4 atomes de carbone, éventuellement substitué par un groupe hydroxyle, R' et R'', pris ensemble, présente le radical bifonctionnel:

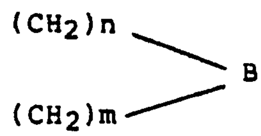

dans lequel:

n et m sont, indépendamment l'un de l'autre, égaux à 2 ou 3; et

B est une liaison directe ou est un atome d'oxygène, de soufre ou un groupe NR''' dans lequel R''' est un atome d'hydrogène ou un radical alkyle linéaire ou ramifié comprenant de 1 à 4 atomes de carbone, éventuellement substitué par un groupe hydroxyle; et

$R_5$ est un groupe nitro, $NH_2$, $NHR_2$ dans lequel $R_2$ est tel que défini ci-dessus ou NHR'''' dans lequel R'''' est un radical présentant la formule suivante:

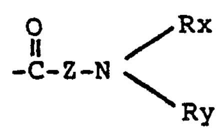

dans laquelle:

Z est un radical alkylène linéaire ou ramifié comprenant de 1 à 4 atomes de carbone; et

$R_x$ et $R_y$ sont, indépendamment l'un de l'autre, un atome d'hydrogène ou un radical alkyle linéaire ou ramifié comprenant de 1 à 4 atomes de carbone, éventuellement substitué par un groupe hydroxy, ou R'''' est le radical:

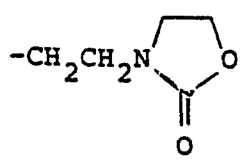

ce procédé consistant a:

à faire réagir un 1-chloro-4-nitro-9H-xanthèn-9-one convenablement substitué et une hydrazine substituée en $R_2$ pour former le composé de formule I, dans lequel $R_5$ est le groupe nitro;

le cas échéant, à convertir ledit composé dans lequel $R_5$ est le groupe nitro par réduction en un composé de formule I, dans lequel $R_5$ est $NH_2$;

le cas échéant encore, à convertir le composé résultant dans lequel $R_5$ est $NH_2$ en un composé de formule I, dans lequel $R_5$ est $NHR_2$ soit (i) par alkylation avec une halo-alkylamine présentant la formule $XR_2$ (éventuellement après protection des groupes sensibles à l'aide de groupes protecteurs), (ii) par réaction

avec un aldéhyde, un acétal, une cétone ou un cétal et réduction de la base de Schiff résultante, ou (iii) par monoacylation à l'aide d'un dérivé réactif d'un agent d'acylation correspondant à R'''' et à réduire le produit acylé (éventuellement après protection des groupes sensibles à l'aide de groupes protecteurs);

le cas échéant, à éliminer les groupes protecteurs par hydrolyse ou réduction et à isoler le produit sous forme de base libre ou sous forme d'un sel d'addition d'acide, dans lequel X est un atome de chlore ou de brome et $R_2$, $R_5$, $R_7$, $R_8$, $R_9$, $R_{10}$ et R'''' sont tels que définis ci-dessus.

2. Un procédé selon la revendication 1, de préparation d'un benzopyran[4,3,2-cd]indazole ayant sous forme de base libre la formule générale suivante:

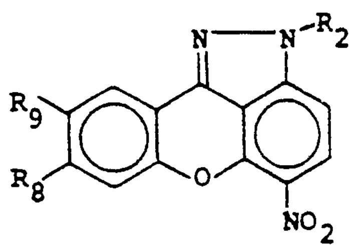

ainsi que les sels pharmaceutiquement acceptables en dérivant, dans laquelle $R_2$, $R_8$ et $R_9$ sont tel que définis dans la revendication 1.

3. Un procédé selon la revendication 1, de préparation d'un benzopyrano[4,3,2-cd]indazole ayant sous forme de base libre la formule générale suivante:

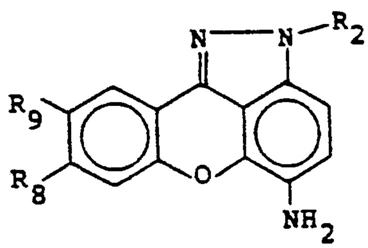

ainsi que les sels pharmaceutiquement acceptables en dérivant, dans laquelle $R_2$, $R_8$ et $R_9$ sont tels que définis dans la revendication 1.

4. Un procédé selon la revendication 1, de préparation d'un benzopyrano[4,3,2-cd]indazole ayant sous forme de base libre la formule générale suivante:

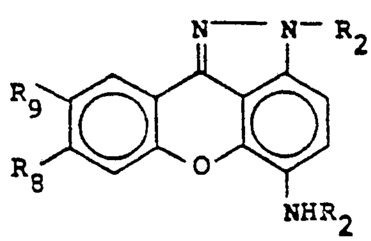

ainsi que le sels pharmaceutiquement acceptables en dérivant, dans laquelle $R_2$, $R_8$ et $R_9$ sont tels que définis dans la revendication 1.

5. Un procédé selon la revendication 1 ou la revendication 4, dans lequel $R_2$ est un radical diéthylaminoéthyl et $R_5$ est $NHCH_2CH_2NH_2$.

6. Un procédé selon la revendication 1, dans lequel on prépare un composé choisi parmi ceux appartenant à la liste suivante:

N-[2-[2-(diéthylamino)éthyl]-2H-[1]-benzopyrano[4,3,2-cd]indazol-5-yl]éthanediamine;

2-[3-(diméthylamino)propyl]-5-nitro-2H-[1]-benzopyrano-[4,3,2-cd]indazol-9-ol; et

2-[2-(diéthylamino)éthyl]-5-[[2-[(2-hydroxyéthyl)amino]éthyl]amino-2H-[1]benzopyrano[4,3,2-cd]indazol-9-ol.

7. Un procédé de préparation d'un composé présentant la formule générale suivante:

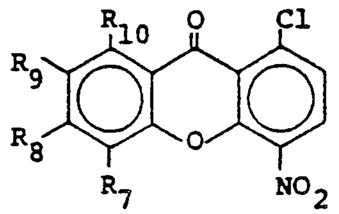

qui consiste à déshydrater de façon non oxydante un composé présentant la formule:

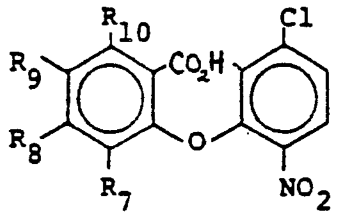

dans laquelle:

$R_7$, $R_8$, $R_9$ et $R_{10}$ sont tels que définis dans la revendication 1.

8. Un procédé selon la revendication 7, dans lequel $R_7$, $R_8$ et $R_{10}$ sont des atomes d'hydrogène.

9. Un procédé de préparation d'une composition pharmaceutique, ce procédé consistant à combiner un composé préparé par un procédé ainsi que revendiqué dans l'une quelconque des revendications 1 à 6 avec un support ou diluant pharmaceutiquement acceptable.